Europäisches Patentamt

European Patent Office

Office européen des brevets

(19)

(11) Publication number: 0 249 854
A2

## (12) EUROPEAN PATENT APPLICATION

(21) Application number: 87108303.6

(22) Date of filing: 09.06.87

(51) Int. Cl.⁴: **C 07 K 13/00**, C 12 N 15/00, C 07 H 21/04, C 12 N 1/20, C 12 N 5/00, C 12 P 21/00, A 61 K 37/02 // (C12N1/20, C12R1:19)

(30) Priority: 10.06.86 JP 134301/86
31.03.87 JP 78187/87

(43) Date of publication of application: 23.12.87
Bulletin 87/52

(84) Designated Contracting States: DE FR GB

(71) Applicant: KYOWA HAKKO KOGYO CO., LTD., 6-1, Ohte-Machi Itchome, Chiyoda-ku Tokyo (JP)

(72) Inventor: Miyaji, Hiromasa, 4-9-14, Naka-machi, Machida-shi Tokyo (JP)
Inventor: Mizukami, Tamio, 1384, Kiso-machi, Machida-shi Tokyo (JP)
Inventor: Mihara, Akira, 1079-22. Kiso-machi, Machida-shi Tokyo (JP)
Inventor: Sato, Moriyuki, 2730-15, Naruse, Machida-shi Tokyo (JP)
Inventor: Itoh, Seiga, 218-14, Aza Hachimannishi Aihara, Sagamihara-shi Kanagawa-ken (JP)

(74) Representative: Casalonga, Axel et al, BUREAU D.A. CASALONGA - JOSSE Morassistrasse 8, D-8000 Munich 5 (DE)

(54) Novel polypeptide.

(57) According to the present invention, a recombinant DNA wherein a DNA coding for a novel physiologically active polypeptide produced by human erythroleukemia cell strain K562-T1 is incorporated and a microorganism or an animal cell carrying the recombinant DNA can be obtained, and they can be utilized for producing a large amount of the physiologically active polypeptide.

EP 0 249 854 A2

## Title of the Invention

Novel polypeptide

### Background of the Invention

The present invention provides a novel physiologically active polypeptide having a cell growth-promoting activity. The substance of the invention is expected to be useful as an immunoactivator and a carcinostatic agent.

So far, the following substances have been known as examples of the substances having a cell growth-promoting activity.

(1)  PDGF (Platelet-derived growth factor) [Proc. Natl. Acad. Sci., USA, 76, 1809 (1979)]

(2)  TGF (Transforming growth factor) [J. Biol. Chem. 257, 5220 (1982)]

(3)  EGF (Epidermal growth factor) [Anal. Biochem. 111, 195 (1981)]

(4)  FGF (Fibroblast growth factor) [Ann. Rev. Biochem. 45, 531 (1976)]

(5)  FSLA (Fibroblast Somatomedin like activity) [J. Cellular Physiology 107, 317 (1981)]

(6)  IGF-I (Insulin like growth factor) [J. Endocr. 85, 267 (1980)]

(7)  IGF-II (Insulin like growth factor) [Diabetes 31, 2823 (1982)]

(8)  MSA (Multiplication stimulating activity) [Nature 272, 776 (1978)]

Among these substances, the substances Nos. 1 - 4, 6 and 7 whose primary structures have been clarified are clearly different from the present substance in N-terminal structure. The other substances have not been identified yet because of difficulty in separating them from the medium components and thus have no practical significance.

Japanese Published Unexamined Patent Application No. 181293/84 discloses a proteinaceous substance having a DNA synthesis-promoting activity produced by a erythroleukemia cell, K562-Tl strain, but the substance is clearly different from the present substance in molecular weight and other properties.

The strain K562-Tl produces a proteinaceous substance having a cell growth-promoting activity upon animal cells besides the said proteinaceous substance having a DNA synthesis-promoting activity. This substance having the cell growth-promoting activity is expected to be useful as an immunoactivator and a carcinostatic agent. However, a process which comprises culturing a human erythroleukemia cell, strain K562-Tl, in a protein-free medium and separating the cell growth-promoting substance from the culture supernatant, followed by purification provides only a limited amount of the substance. Thus, it has been desired to develop a process for providing the proteinaceous substance having the cell growth-promoting activity in a large amount at a low cost.

The present inventors have made studies of a process for producing a physiologically active (active in cell growth promotion) polypeptide which is produced by a human erythroleukemia cell, strain K562-Tl, using recombinant DNA techniques. As the result, the inventors have succeeded in preparation of a DNA complementary to the physiologically active polypeptide, a recombinant DNA containing the said DNA and a microorganism and an animal cell containing the recombinant DNA, which are applicable to the production of the physiologically active polypeptide. That is, messenger RNA (mRNA) was extracted from the human erythroleukemia cell, strain K562-Tl, and a DNA complementary thereto (cDNA) was synthesized.

On the other hand, the physiologically active polypeptide was separated from the supernatant obtained by protein-free culturing of the human erythroleukemia cell, strain K562-Tl, and purified, and the sequence of polypeptide on the N-terminal portion was determined up to the 30th amino acid. Then, a DNA probe corresponding to the amino acid sequence was synthesized, and cDNA hydridizable with the DNA was selected. The cDNA was incorporated into a vector, and expressed in animal cells, and cDNA showing the physiological activity was selected.

Thus, cDNA containing the gene coding for the novel physiologically active polypeptide produced by the human erythroleukemia cell, strain K562-Tl was obtained. Then, the entire nucleotide sequence of this cDNA was determined.

Further, the present inventors have found that the physiologically active polypeptide can be prepared by modifying the physiologically active polypeptide cDNA shown in Tables 2, 3 and 6, introducing the cDNA into Escherichia coli or an animal cell and culturing the Escherichia coli or animal cell, and has established the present invention.

Ubiquitin recovered from a calf thymus can be mentioned as a substance similar to the present substance. The amino acid sequence from the N-terminal to the 30th amino acid of the present substances shown in Tables 2 and 3 is substantially the same as the sequence of ubiquitin. The present substance shown in Table 6 has a structure wherein three ubiquitin monomers are combined with one another in series and cysteine is attached to the C-terminal. Ubiquitin shows no growth-promoting activity upon Namalwa cells, and thus the present substances are clearly different from ubiquitin. Histone protein-fixed ubiquitin, precursor composed of ubiquitin molecules combined with one another in series, etc. are known [Nature 225, 423 (1975); JBC 250, 7182 (1975); Nature 312, 663 (1984)]. The biological activities of these substances have not been clarified yet.

Recently, cloning of human ubiquitin precursor gene has been reported [P.K. Lund, et al.: J. Biol. Chem. 260, 7609 (1985); O. Wiborg, et al.: EMBO J. 4, 755 (1985)]. The human ubiquitin precursor cloned by Lund, et al. has a structure wherein 9 ubiquitin monomers are combined with one another in series and is clearly different from the present substance. The human ubiquitin precursor cloned by Wiborg, et al. is deficient in the nucleotide sequence for the N-terminal 4 amino acids in contrast to the present substance, and no precursor containing the nucleotide sequence coding for the amino acids corresponding thereto has been recovered. Thus, the precursor is clearly different from the present substance.

## Summary of the Invention

The present invention provides a polypeptide having the peptide sequence shown in Table 2, 3 or 6, which can be prepared in the following manner using recombinant DNA techniques.

With mRNA of a physiologically active polypeptide produced by the strain K562-T1 as a template, a DNA (cDNA) showing complementarity to the mRNA is prepared, and a recombinant plasmid wherein the cDNA is incorporated into a vector plasmid is prepared. Then, the recombinant plasmid is introduced into a host microorganism or a host animal cell. The DNA and the recombinant plasmid can be used to amplify the physiologically active polypeptide gene in bacteria such as Escherichia coli.

Any plasmid can be used as a plasmid for incorporating the DNA coding for the physiologically active polypeptide, so long as it can express the DNA in Escherichia coli or an animal cell. Microorganisms and animal cells carrying the recombinant plasmid are useful for producing the physiologically active polypeptide in a large amount at a low cost.

Thus, the present invention also provides a DNA coding for the physiologically active polypeptide produced by the strain K562-T1, a recombinant plasmid wherein the DNA is incorporated, a microorganism and an animal cell containing the plasmid and a process for producing them.

The invention further relates to a medicament containing as an active ingredient a polypeptide according to the invention as well as to a pharmaceutical composition containing such a polypeptide. The pharmaceutical composition may be used as an immunoactivating or carcinostatic agent.

In addition, the invention relates to the use of a polypeptide according to the invention or of a composition containing such polypeptide for the manufacture of a medicament having a growth promoting activity upon human B lymphocytic cells. Said medicament may be used as an immunoactivating or carcinostatic agent.

Brief Description of the Drawings

Fig. 1 shows the outline of cDNA synthesis according to the method of Okayama-Berg and a process for constructing a recombinant plasmid containing the DNA.

Fig. 2 shows restriction enzyme maps of the cDNAs contained in pLGA8, pLGB12 and pLGC15.

Fig. 3 is a flow sheet showing a process for constructing plasmid pLGB-al.

Fig. 4 is a flow sheet showing a process for constructing plasmid pLGB-d2.

Fig. 5 is a flow sheet showing a process for constructing plasmid pLGA-d5.

Fig. 6 is a flow sheet showing a process for constructing plasmid pLGA-el.

Fig. 7 is a flow sheet showing a process for constructing plasmid pLGC:Scll.

Fig. 8 is a flow sheet showing a process for constructing plasmid pLGC-xl.

Fig. 9 is a flow sheet showing a process for constructing plasmid pSEA-el.

Detailed Description of the Invention

The DNA and the recombinant plasmid of the present invention can be prepared according to the following general procedures.

Whole RNAs are prepared from the strain K562-Tl, and RNA having polyadenylic acid (polyA) [poly(A)RNA] is separated therefrom by passing the whole RNAs through a column of oligo dT cellulose. With the poly(A)RNA as a template, a double-stranded DNA is synthesized by means of a reverse transcriptase. A recombinant can be obtained by inserting the double-stranded DNA into a vector DNA such as a plasmid DNA of Escherichia coli using in vitro recombinant DNA techniques. A recombinant plasmid having a DNA complementary to the physiologically active polypeptide mRNA is selected.

A process for producing the DNA and the recombinant plasmid of the present invention is described in detail below.

Cells are collected from a suspension of K562-Tl cells by centrifugation, and solubilized in a solution containing guanidium thiocyanate. Then, the solubilized product is laid on a layer of CsCl solution, and, after ultracentrifugation, whole cytoplasmic RNAs are obtained as precipitates. Alternatively, LiCl is added to the guanidium thiocyanate-solubilized product to recover only RNAs by precipitation.

The precipitated RNAs are dissolved in a solution of NaCl or KCl at a high salt concentration (e.g. 0.5 M) and the solution is passed through a column of oligo(dT) cellulose to adsorb mRNA having poly(A) on the column. mRNA having poly(A) is isolated by elution with water or a solution of low salt concentration such as 10 mM Tris-HCl buffer.

Then, synthesis of cDNA and incorporation thereof into a vector are carried out according to Okayama-Berg procedure [Okayama & Berg; J. Mol. Cell. Biol. $\underline{2}$, 161 (1982)].

First, a vector primer is synthesized. A vector, e.g. pcDVl is treated with KpnI in an appropriate solution, e.g. a solution containing Tris-HCl buffer (e.g. pH 7.5, 10 mM), $MgCl_2$ (e.g. 6 mM) and NaCl (e.g. 10 mM) to cleave pcDVl at the KpnI site. The DNA is incubated together with terminal deoxynucleotidyl transferase in a solution containing Tris-HCl buffer (e.g. pH 6.8, 30 mM), sodium cacodylate (e.g. 140 mM), $CoCl_2$ (e.g. 1 mM), dithiothreitol (e.g. 0.1 mM) and dTTP (e.g. 0.25 mM) at a constant temperature (e.g. 37°C) for a predetermined period (e.g. 20 minutes) to attach about 60 thymidyl residues to both 3'-ends of the vector DNA. The DNA is cleaved with EcoRI in a solution containing Tris-HCl buffer (e.g. pH 7.5, 10 mM), $MgCl_2$ (e.g. 6 mM), and NaCl (e.g. 100 mM), and fractionated by low gelling temperature agarose gel electrophoresis [Lars Wieslander: Analytical Biochemistry, $\underline{98}$, 305 (1979), hereinafter referred to as LGT method] to recover a fragment of about 3.1 kilobases. Then, the DNA is dissolved in a solution of NaCl or KCl at a high salt concentration (e.g. 0.5 M) and the solution is passed through a column of poly(dA) cellulose to adsorb only vector primer molecules having poly(T) on the column. Poly(T)-attached vector primer molecules are isolated by elution with water or a solution of low salt concentration such as 10 mM Tris-HCl buffer.

Then, a linker DNA is synthesized. For example, pL1 DNA is treated with PstI in an appropriate solution such as a solution containing Tris-HCl buffer (e.g. pH 7.5, 10 mM), $MgCl_2$ (e.g. 6 mM), and NaCl (e.g. 50 mM) to cleave pL1 at the PstI site. The DNA is treated in the same manner as in the synthesis of vector primer except that dGTP is added in place of dTTP, whereby olido dG chains (about 15 mer) are added. The DNA is cleaved with HindIII in an appropriate solution such as a solution containing Tris-HCl buffer (e.g. pH 7.5, 10 mM), $MgCl_2$ (e.g. 6 mM) and NaCl (e.g. 60 mM). A DNA fragment of about 0.5 kilobase is fractionated by agarose gel electrophoresis, and recovered on DEAE paper, whereby the linker DNA can be obtained.

The thus obtained poly(A)RNA, vector primer and linker DNA are used to synthesize cDNA. The poly(A)RNA and vector primer DNA are made to react with a reverse transcriptase in a solution containing Tris-HCl buffer (e.g. pH 8.3, 50 mM), $MgCl_2$ (e.g. 8 mM), KCl (e.g. 30 mM), dithiothreitol (e.g. 0.3 mM), and dATP, dTTP, dCTP and dGTP (e.g. 2 mM each) at a constant temperature (e.g. 37°C) for a predetermined period (e.g. 40 minutes). Oligo dC chains (about 15 mer) are attached to the 3'-ends of the thus obtained RNA-DNA double stranded chain in the same manner as in the case of attaching the dT chains to the vector primer except that dTTP is replaced with dCTP. The DNA is cleaved with HindIII in a solution containing Tris-HCl buffer (e.g. pH 7.5, 10 mM), $MgCl_2$ (e.g. 6 mM) and NaCl (e.g. 60 mM). The DNA is mixed with the previously prepared linker DNA, and incubated together with Escherichia coli DNA ligase in a solution containing Tris-HCl buffer (e.g. pH 7.5, 20 mM), MgCl$_2$ (e.g. 4 mM), (NH$_4$)$_2$SO$_4$ (e.g. 10 mM), KCl (e.g. 0.1 M) and β-nicotinamide adenine dinucleotide (β-NAD) (e.g. 0.1 mM) at a constant temperature (e.g. 12°C) for a predetermined period (e.g. 16 hours), whereby cyclization of cDNA and linker DNA is carried out. To the reaction solution are added 40 μM (final concentration) each dATP, dTTP, dGTP and dCTP. Then, Escherichia coli DNA ligase, Escherichia coli DNA polymerase I and Escherichia coli ribonuclease(RNase)H are added thereto to convert the RNA moiety to DNA, whereby a recombinant plasmid containing a complete double-stranded cDNA can be obtained.

An Escherichia coli strain, for example, Escherichia coli C600 SF8 strain [Cameron, et al.: Proc. Natl. Acad. Sci., USA, 72, 3416 (1975)] is subjected to transformation with the thus obtained recombinant plasmid, for example, by the method of Scott, et al. [Katsuya Shigesada: Saibo Kogaku (Cell Engineering), 2, 616 (1983)]. Since there are ampicillin-resistance genes on the recombinant plasmid obtained above, the transformed Escherichia coli shows resistance to ampicillin.

On the other hand, the physiologically active polypeptide can be prepared according to the process shown in Reference Example 1 to determine its amino acid sequence. Any human erythroleukemia cell can be used in the present invention, so long as it can produce the present substance. Preferably, erythroleukemia cell K562-T1 strain is used.

The K562-T1 strain [T. Okabe, et al.: Proc. Natl. Acad. Sci., USA, 81, 453 (1984)] is a protein-free medium-habituated cell obtained by subjecting K562 strain (ATCC CCL 243) to habituation in Ham F10 medium containing fetal calf serum for about one year while gradually lowering the serum concentration from the initial concentration of 10%. The K562 strain is a well known cell strain disclosed in Proc. Natl.

Acad. Sci., USA, 76, 1293 (1979); Blood. 45, 321 (1975); GANN, 73, 97 (1982), etc. and is generally available.

As the medium, protein-free media such as Ham F10 medium, Ham F12 medium (these two media are made by Flow Laboratory, Inc.), Dulbecco's Modified Eagle (DME) medium, MEM medium, RPMI-1640 medium (these three media are made by Nissui Seiyaku K.K., Japan), etc., and their mixed media can be used. The medium can contain, if necessary, appropriate amounts of glutamine (0.5 - 5 mM), antibiotics [penicillin (25 U/m$\ell$), streptomycin (25 µg/m$\ell$), etc.], sodium bicarbonate (0.01%), etc.

For the culturing, various culturing bottles, dishes, roller bottles, spinner flasks, jar fermentors, etc. can be used. By culturing usually at a seed cell density of $5 \times 10^4$ to $1 \times 10^6$ cells/m$\ell$ at 30 to 40°C for 2 to 4 days, the present substance is produced mainly in the culture liquor according to the cell density. For example, culturing at a seed cell density of $1 \times 10^5$ cells/m$\ell$ and at 37°C for 2 days can produce 300 units/m$\ell$ active substance in the culture liquor.

The physiologically active polypeptide can be recovered from the culture liquor in the following manner. That is, the supernatant of the obtained culture liquor is concentrated by freeze-drying, ultrafiltration, strongly acidic ion exchange resin treatment, etc. For elution, a weakly basic buffer or an alkali solution at a low concentration is used.

The concentrated solution contains many low molecular weight salts and impurities and thus is dialyzed against 1% acetic acid, whereby most of impure proteins are removed as precipitates. The acetic acid is removed by freeze-drying, and then the solution is further concentrated to obtain a crude product. The crude product is passed through a column of cation exchange resin. There exists only a weak interaction between the active substance and the cation exchange resin, and the active substance is eluted without being adsorbed on the resin. The active fractions are collected and concentrated by freeze-drying, etc., and the low molecular weight substances originating from the medium are removed by gel filtration. As the gel filtration medium, Sephadex (made by Pharmacia Fine Chemicals Inc.), Biogels (made by Bio-Rad Laboratories), Control pore glass (made by Corning Glass Works), Toyopearl (made by Toyo Soda), etc. are used.

When Biogel P-60, or the like is used, the active fraction is found between the void volume (V0) and the low molecular weight substances such as salts, etc.

The concentrate obtained through the foregoing operations can be purified as a single component by high pressure liquid chromatography.

The amino acid sequence of the thus obtained physiologically active polypeptide can be determined by a combination of, for example, Type 470A sequencer made by Applied Biosystems and a high pressure liquid chromatography made by Spectra Physics. As a result of the determination of the polypeptide sequence on the N-terminal portion up to the 30th amino acid, it has been found that the amino acid sequence from the N-terminal to the 30th amino acid of the physiologically active peptide are substantially the same as the sequence of human ubiquitin.

A strain containing a novel recombinant plasmid DNA having genes complementary to the physiologically active polypeptide mRNA can be selected from the ampicillin-resistant (Ap$^r$) strains by the following generally employed method. That is, the transformants obtained by the above-described method are immobilized on a nitrocellulose filter, and are hybridized with a synthetic DNA probe having the DNA sequence expected from the amino acid sequences of known physiologically active protein produced by the K562-Tl strain and human ubiquitin, and the strongly hybridized strain is selected [the method of Grunstein-Hogness: Proc. Natl. Acad. Sci., USA, 72, 3961 (1975)]. The probe DNA is synthesized according to the ordinary triester method [J. Am. Chem. Soc., 97, 7327 (1975)]. The selection by the synthetic DNA can be further ensured by the method of Southern, et al. [J. Mol. Biol., 98, 503 (1975)]. Identification of the recombinant plasmid DNA having a gene complementary to the physiologically active polypeptide mRNA is carried out by introducing the plasmid into an animal cell such as COS-1 cell ATCC CRL 1650 [Y. Gluzman: Cell 23, 175 (1981)] by, for example, the DEAE-dextran method [L.M. Sompayrac, et al.: Proc. Natl. Acad. Sci., USA, 78, 7575 (1981)] to express the gene, disrupting the cell in Teflon homogenizer, dialyzing the cell extract against a phosphate buffer, and measuring the cell growth-promoting activity. The cell growth-promoting activity can be measured in the following manner using a human B lymphocytic cell such as human lymphoblastoid cell strain Namalwa (ATCC CRL 1432).

For example, 5 to 10 x $10^5$ cells/mℓ the test cell strain is cultured in 75 mℓ of RPMI-1640 medium (made by Nissui Seiyaku K.K., Japan) containing glutamine (4 mM), streptomycin (25 μg/mℓ), penicillin (25 U/mℓ), N-2-hydroxyethylpiperazine-N'-2-ethanesulfonic acid (HEPES) (10 mM), sodium bicarbonate (0.01%) and calf serum (10%) at 37°C for 48 hours. The culture broth is centrifuged under 800 x G for 5 minutes to collect cells, and the cells are washed with the same medium as the said medium only free from the serum, and poured onto a 24-well multidish plate. 0.9 mℓ of the serum-free medium and 0.1 mℓ of the sample solution are added thereto, and after culturing at 37°C for 3 days in a $CO_2$ incubator, the number of cells is counted with a hemocytometer.

The physiologically active polypeptide can be expressed by incorporating a DNA fragment coding for the physiologically active polypeptide into an appropriate plasmid having a function to express DNAs.

As the DNA coding for the physiologically active polypeptide shown in Table 2, 3 or 6, cDNA obtained from messenger RNA coding for the physiologically active polypeptide by reverse transcription according to the recombinant DNA technology (the physiologically active polypeptide cDNA), DNA coding for the physiologically active polypeptide obtained from chromosomal DNA, etc. can be utilized.

As the physiologically active polypeptide cDNA, any cDNA can be used so long as it codes for the physiologically active polypeptide, and specifically, pLGA8, pLGB12 and pLGC15 can be used. pLGA8, pLGB12 and pLGC15 are those prepared by the present inventors, and the processes for preparing them are disclosed in Examples. The physiologically active polypeptide DNAs in pLGA8, pLGB12 and pLGC15 have the nucleotide sequences shown in Tables 2, 3 and 6 determined by the dideoxy sequence method [J. Messing, et al.: Gene 19, 269 (1985)] using M13 phage.

As the plasmid for incorporating a DNA coding for the physiologically active polypeptide, any plasmid can be used so long as it can express the DNA in Escherichia coli or an animal cell. Preferably, a plasmid wherein a foreign DNA can be inserted at a position downstream from an appropriate promoter such as trp promoter or lac promoter and the length between the Shine-Dalgarno sequence (hereinafter referred to as SD sequence) and the initiation codon (ATG) is appropriately adjusted, for example, to 6 - 18 base pairs is employed. As specifically preferable plasmids, pKYP10, pKYP11 and pKYP12 (Japanese Published Unexamined Patent Application No. 110600/83) constructed by the present inventors, and the like can be mentioned.

An example of construction of a recombinant plasmid containing a DNA coding for the physiologically active polypeptide is described below, wherein pLGA8, pLGB12 and pLGC15 are used as the physiologically active polypeptide cDNAs and pKYP10 as the plasmid for incorporating the cDNA.

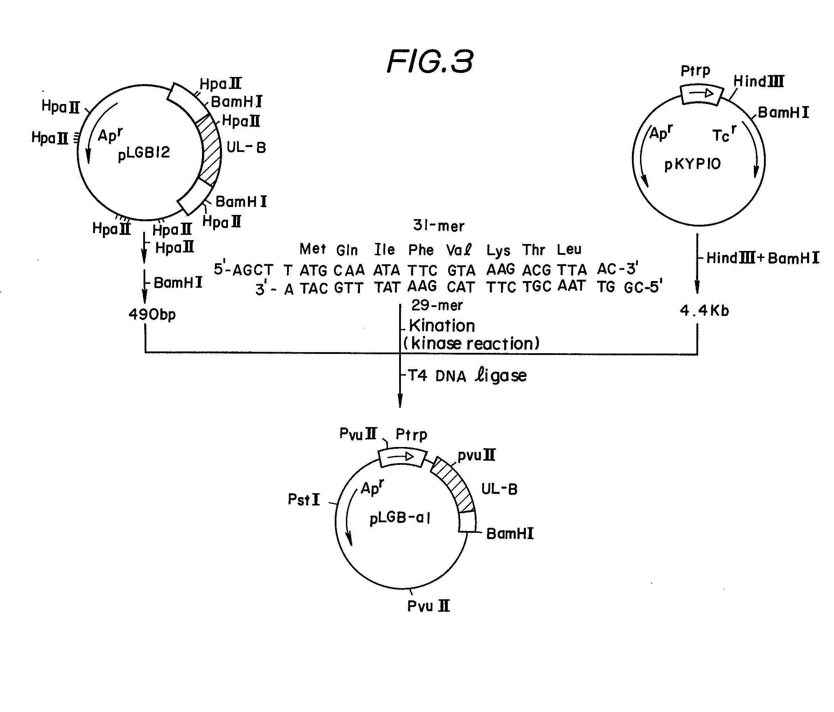

As shown in Fig. 3, pLGB12 is cleaved with HpaII and BamHI, and DNA fragments of about 490 base pairs (hereinafter referred to as bp) are purified by LGT method. pKYP10 is cleaved with HindIII and BamHI, and DNA fragments of about 4.4 kilobases (hereinafter referred to as Kb) are purified by LGT method. The thus obtained DNA fragments are combined with a synthetic DNA shown in Fig. 3, which includes up to the second base (AC) of triplet (ACC) coding for threonine (Thr) as the 9th amino acid from the initiation codon (ATG), using T4 DNA ligase to obtain pLGB-al. In the Figure, UL-B is the DNA region shown in Table 3.

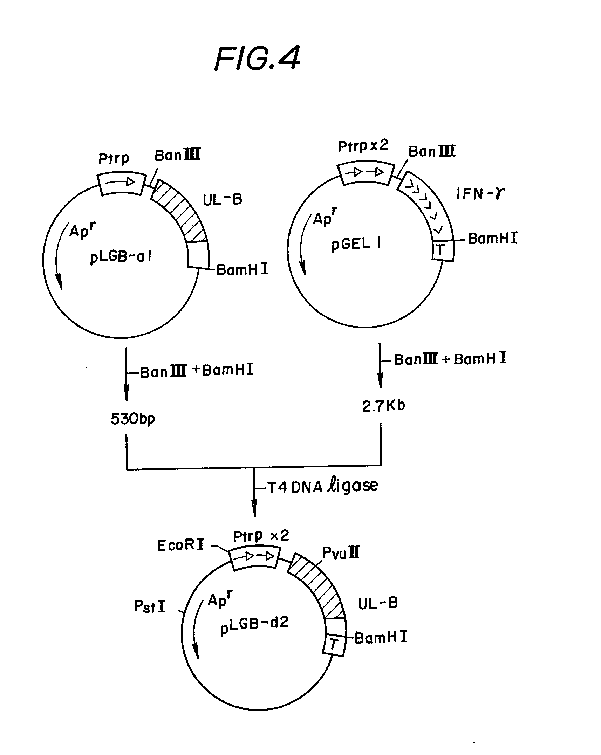

Then, pLGB-al is cleaved with BanIII and BamHI as shown in Fig. 4, and DNA fragments of about 530 bp are purified by LGT method. pGEL1 [Sekine, et al.: Proc. Natl. Acad. Sci., USA, 82, 4306 (1985)] is also cleaved with BanIII and BamHI, and DNA fragments of about 2.7 Kb are purified by LGT method.

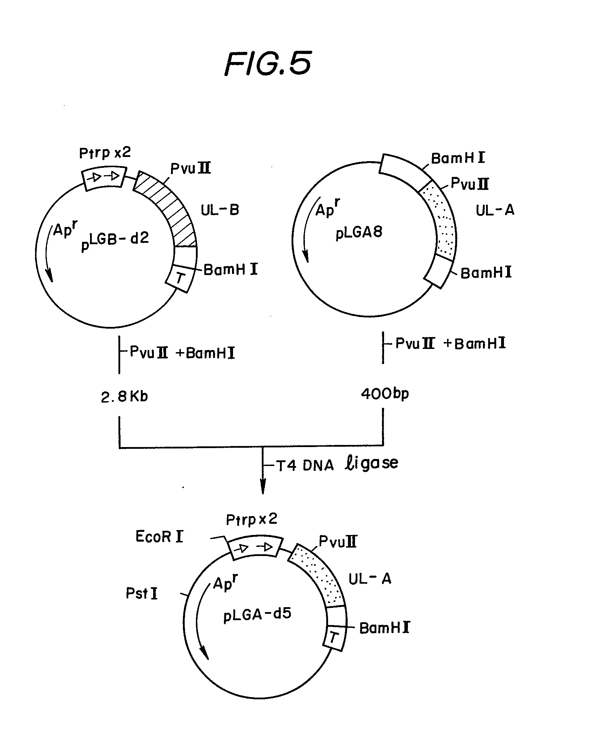

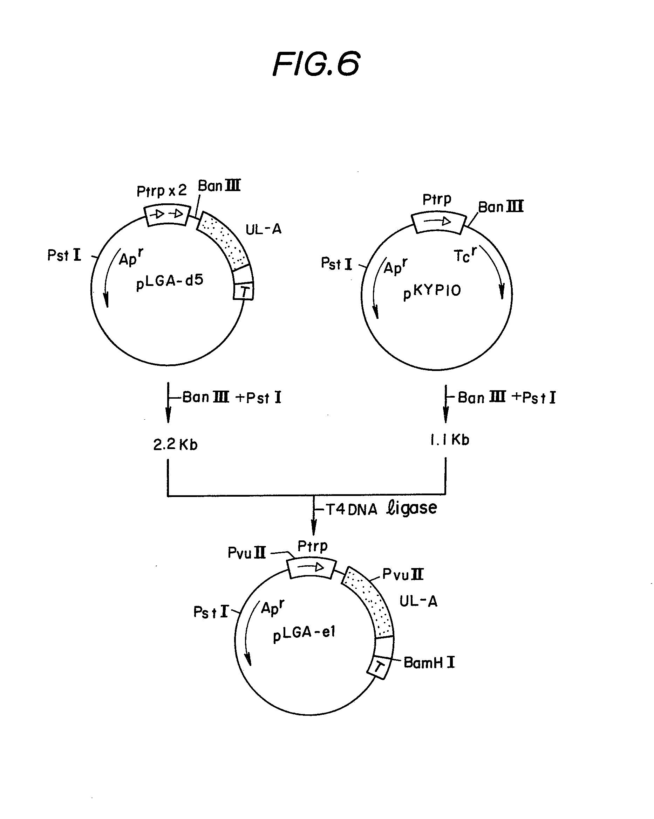

The thus obtained DNA fragments are combined together with T4 DNA ligase to obtain pLGB-d2. Then, pLGB-d2 is cleaved with PvuII and BamHI as shown in Fig. 5, and DNA fragments of about 2.8 Kb are purified. pLGA8 is also cleaved with PvuII and BamHI, and DNA fragments of about 400 bp are purified. The thus obtained DNA fragments are combined together with T4 DNA ligase to obtain pLGA-d5. Then, pLGA-d5 is cleaved with BanIII and PstI as shown in Fig. 6, and DNA fragments of about 2.2 Kb are purified. On the other hand, pKYP10 is cleaved with BanIII and PstI, and DNA fragments of about 1.1 Kb are purified. The thus obtained DNA fragments are combined together with T4 DNA ligase to obtain pLGA-el. In the Figure, UL-A is the DNA region shown in Table 2.

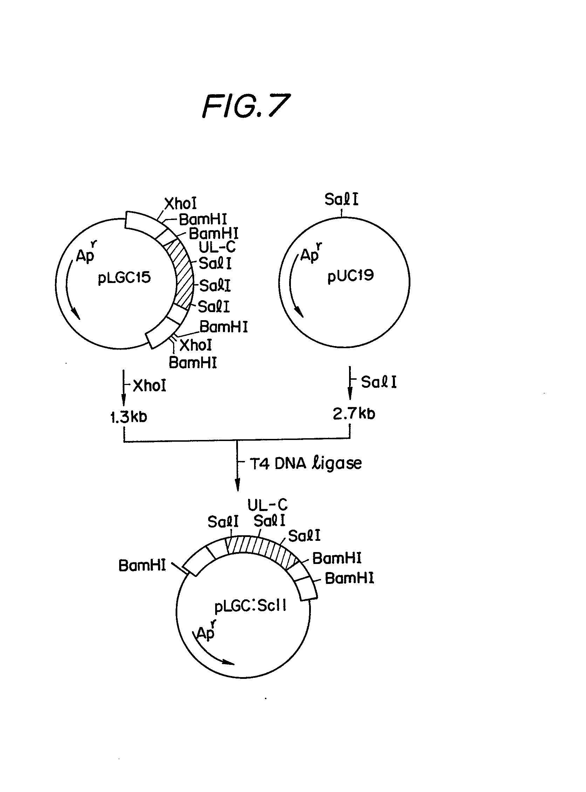

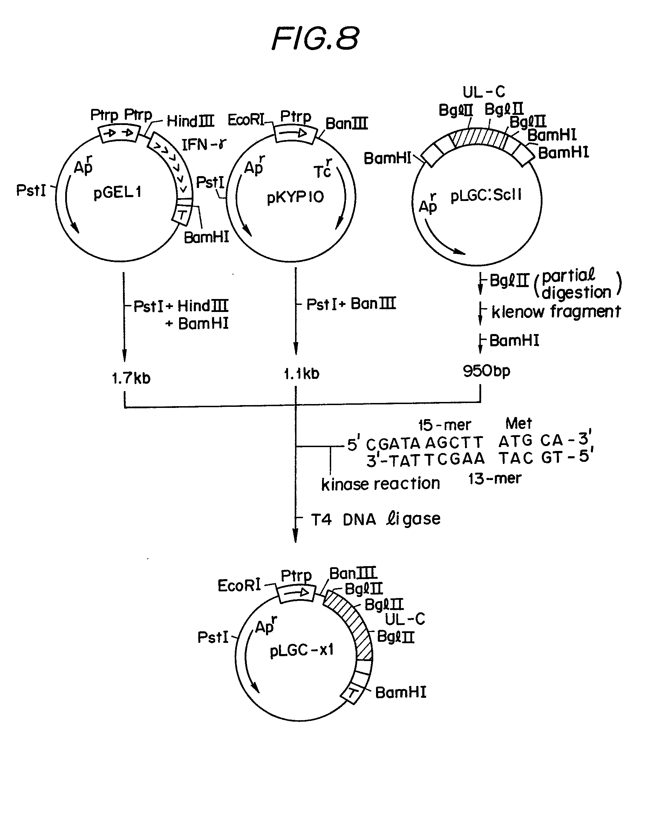

Then, as illustrated in Fig. 7, pLGC15 is cleaved with XhoI, and DNA fragments of about 1.3 Kb are purified by LGT method. pUC19 [C. Yanisch-Perron, et al.: Gene 33, 103 (1985)] is cleaved with SalI, and DNA fragments of about 2.7 Kb are purified. The thus obtained DNA fragments are

ligated with T4 DNA ligase to obtain pLGC:Sc11. Further, as illustrated in Fig. 8, pLGC:Sc11 is partially digested with BglII, treated with DNA polymerase I·Klenow fragment (referred to as Klenow fragment hereinafter), cleaved with BamHI and subjected to purification by LGT method to obtain DNA fragments of about 950 bp.

pGEL1 is cleaved with BamHI, HindIII and PstI, and DNA fragments of about 1.7 Kb are obtained by LGT method. Separately, pKYP10 is cleaved with PstI and BanIII, and DNA fragments of about 1.1 Kb is purified by LGT method. The thus obtained DNA fragments and the synthetic DNA, which is illustrated in Fig. 8 and contains up to the second base (CA) of triplet (CAG) coding for glutamine (Gln) as the second amino acid from the initiation codon (ATG), are ligated with T4 DNA ligase to obtain pLGC-x1. In the Figure, UL-C indicates the DNA region illustrated in Table 6.

Reaction conditions required for the recombinant DNA techniques described above are generally as follows.

Digestion of the DNA with restriction enzymes is usually carried out by reacting 0.1 to 20 μg of the DNA with 0.1 - 100 units of the restriction enzyme, preferably 1 - 3 units of the restriction enzyme per 1 μg of the DNA in a mixture of 2 - 200 mM, preferably 10 - 40 mM Tris-HCl (pH 6.0 - 9.5, preferably pH 7.0 - 8.0), 0 - 200 mM NaCl and 2 - 20 mM, preferably 5 - 10 mM $MgCl_2$ at 20 - 70°C (optimal temperature depends on restriction enzymes used) for 15 minutes to 24 hours. Reaction is usually stopped by heating at 55 - 75°C for 5 - 30 minutes, or alternatively by inactivating the restriction enzyme with a reagent such as phenol or diethylpyrocarbonate.

Purification of the DNA fragments formed by digestion with restriction enzymes is carried out by the above-mentioned LGT method or polyacrylamide gel electrophoresis.

Ligation of the DNA fragments is carried out with 0.3 - 100 units of T4 DNA ligase in a mixture of 2 - 200 mM, preferably 10 - 40 mM Tris-HCl (pH 6.1 - 9.5, preferably 7.0 - 8.0), 2 - 20 mM, preferably 5 - 10 mM $MgCl_2$, 0.1 - 10 mM, preferably 0.5 - 2.0 mM ATP and 1 - 50 mM, preferably 5 - 10 mM dithiothreitol at 1 - 37°C, preferably 3 - 20°C for 15 minutes to 72 hours, preferably, 2 - 20 hours.

The recombinant plasmid DNA formed by the ligation reaction is introduced into Escherichia coli by the transformation method of Cohen, et al. [S.N. Cohen, et al.: Proc. Natl. Acad. Sci. USA, 69, 2110 (1972)], if necessary.

Isolation of the recombinant plasmid DNA from Escherichia coli carrying the DNA is carried out by the method described in Example 1 or the method of Birnboim, et al. [H.C. Birnboim, et al.: Nucleic Acids Res. 7, 1513 (1979)].

Plasmid DNA is digested with 1 - 10 kinds of restriction endonucleases and the cleavage sites are examined by agarose gel electrophoresis or polyacrylamide gel electrophoresis. Further, if necessary, the nucleotide sequence of the DNA is determined by the dideoxy sequence method.

The recombinant plasmid DNAs can be prepared under the above-described conditions.

The physiologically active polypeptide of the present invention is produced by the following method.

That is, Escherichia coli C600 SF8 is transformed with a plasmid such as pLGB-al and an Escherichia coli strain carrying pLGB-al is selected from the ampicillin resistant (referred to as Ap^r hereinafter) colonies. The Escherichia coli strain carrying pLGB-al is cultured in a medium to produce the physiologically active polypeptide in the culture broth.

As the medium, either a synthetic medium or a natural medium can be used as long as it is suitable for the growth of Escherichia coli and the production of the physiologically active polypeptide.

As the carbon source, glucose, fructose, lactose, glycerol, mannitol, sorbitol, etc. may be used. As the nitrogen source, $NH_4Cl$, $(NH_4)_2SO_4$, Casamino acids, yeast extract, polypeptone, meat extract, Bacto-Tryptone, corn steep liquor, etc. may be used. In addition, nutrients such as $K_2HPO_4$, $KH_2PO_4$, NaCl, $MgSO_4$, vitamine $B_1$ and $MgCl_2$ may be used.

Culturing is carried out at pH 5.5 - 8.5 and at 18 - 40°C with aeration and stirring. After culturing for 5 - 90 hours, the physiologically active polypeptide accumulates in cultured cells. The cells are collected from the culture broth and disrupted by ultrasonic treatment. By centrifugation, the physiologically active polypeptide is separated and extracted into the supernatant. After removal of the cells with a 0.45-μm filter, the cell growth-promoting activity can be determined according to the said method using human B lymphocytic cells such as human lymphoblastoid cell strain Namalwa.

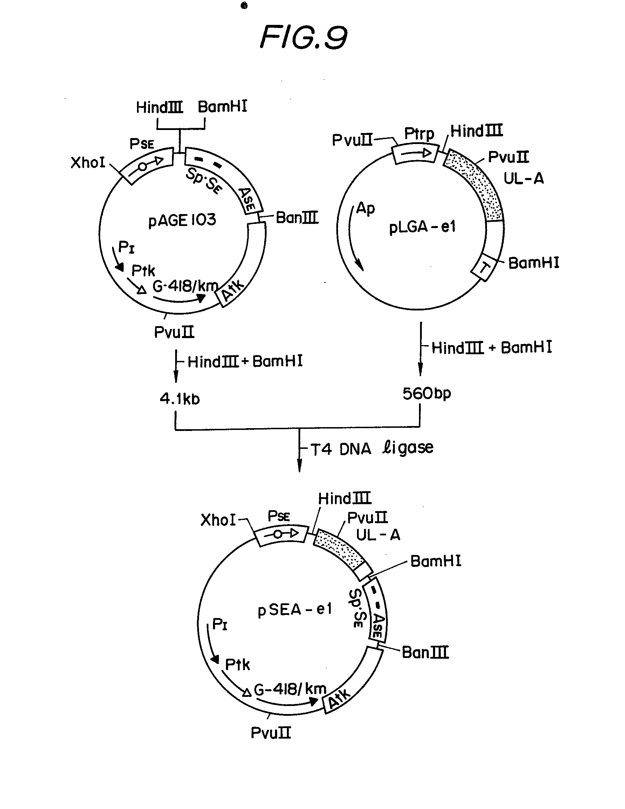

As the plasmid for expressing the DNA coding for the physiologically active polypeptide in animal cells, any plasmid can be used so long as it can express the DNA in animal cells. Preferably, a plasmid wherein a foreign DNA can be inserted downstream from a suitable promoter such as SV40 early promoter or SV40 late promoter and which has poly(A) signal, splicing signal and the like is used. As the preferable plasmids, pAGE103 constructed by the present inventors and the like are mentioned. pAGE103 is a plasmid illustrated in Fig. 9 and Escherichia coli carrying the plasmid was deposited with the Fermentation Research Institute, Agency of Industrial Science and Technology (FRI) as Escherichia coli EAGE103 (FERM BP-1312) on March 23, 1987. As the plasmid containing dihydrofolate reductase (referred to as dhfr hereinafter) gene as a selection marker, pSV2-dhfr [S. Subramani, et al.: Mol. Cell. Biol. 1, 854 (1981)] and the like are mentioned.

As the host for the expression of the physiologically active polypeptide, any animal cell can be used so long as the peptide is expressed in the cell. As the preferable examples of animal cells, CHO cell deficient in dhfr [G. Vrlaub & L.A. Chasin: Proc. Natl. Acad. Sci. USA, 77, 4216 (1980)] and the like are mentioned.

An example of a process for producing the physiologically active peptide is described below, wherein a recombinant plasmid which can express the peptide in an animal cell is constructed using pLGA-el [Escherichia coli carrying pLGA-el was deposited with the FRI as Escherichia coli ELGA-el (FERM BP-1313) on March 23, 1987] as the cDNA coding for the peptide and pAGE103 as the plasmid for incorporating the cDNA and the peptide is produced using plasmid pSV2-dhfr containing dhfr gene as the selection marker and CHO cells deficient in dhfr as the host.

As illustrated in Fig. 9, pAGE103 is cleaved with HindIII and BamHI, and DNA fragments of about 4.1 Kb are purified by LGT method. pLGA-el is cleaved with HindIII and BamHI, and DNA fragments of about 560 bp are purified by LGT method. The thus obtained DNA fragments are ligated with T4 DNA ligase to obtain pSEA-el. Reaction conditions employed in the recombinant techniques are the same as those described above.

The physiologically active polypeptide of the present invention is produced by the following method. That is, plasmids pSEA-el and pSV2-dhfr are introduced into CHO strain deficient in dhfr by, for example, calcium phosphate method [Graham & Van der Eb: Virology 52, 546 (1978)]. Transformants carrying both pSEA-el and pSV2-dhfr are selected, for example, on MEM ALPHA medium containing G418 and dialyzed fetal calf serum (product of Gibco Oriental; free of ribonucleic acids and deoxyribonucleic acids). Further, transformants which contain amplified physiologically active polypeptide gene can be selected by using methotrexate. The thus obtained transformant is cultured in a medium to produce the physiologically active polypeptide in the culture broth.

As the medium, Ham F10 medium, Ham F12 medium (products of Flow Laboratory Inc.), Dulbecco's Modified Eagle (DME) medium, RPMI-1640 medium (products of Nissui Seiyaku) and MEM ALPHA medium, which contain various sera such as fetal calf serum, and mixtures thereof are used. If necessary, glutamine (0.5 - 5 mM), antibiotics [penicillin (25 U/mℓ), streptomycin (25 µg/mℓ), G418 (0.3 mg/mℓ), etc.], sodium bicarbonate (0.01%) and the like may be added in an appropriate amount to the medium.

For the culturing, various culture bottles, dishes, roller bottles, spinner flasks, jar fermentors and the like can be used. By culturing usually at a seed cell density of 5 x $10^4$ to 1 x $10^6$ cells/mℓ at 30 - 40°C for 2 - 10 days, the substance of the present invention is produced mainly in the cells according to the cell density.

Cells are recovered from the culture broth by centrifugation and disrupted by, for example, using Teflon homogenizer, and the physiologically active polypeptide is extracted in a supernatant by centrifugation. Cell growth-promoting activity is determined by the method mentioned above using human B lymphocytic cells such as human lymphoblastoid cell strain Namalwa.

The novel recombinant plasmid of the present invention can be used for producing a large amount of the physiologically active polypeptide by a microorganism such as Escherichia coli or a eukaryotic cell.

Certain specific embodiments of the invention are illustrated by the following representative examples.

Example 1

Preparation of poly(A)RNA from K562-T1 strain:

RNA having poly(A) was prepared from a human erythroleukemia cell strain, K562-T1 strain according to guanidium thiocyanate-lithium chloride method [Cathala, et al.: DNA 2, 329 (1983)] in the following manner.

The K562-T1 strain was inoculated into 1 ℓ of a protein-free medium prepared by mixing Ham F10 medium (made by Flow Laboratory Inc.) with Dulbecco's Modified Eagle (DME) medium (made by Nissui Seiyaku) in a mixing ratio of 3:1 to make a basal medium, and adding pyruvic acid (5 mM), selenious acid (1.25 x 10$^{-7}$ M), galactose (1 mg/mℓ), glutamine (4 mM), penicillin (25 U/mℓ), streptomycin (25 µg/mℓ) and sodium bicarbonate (0.01%) to the basal medium to make a density of 8 x 10$^5$ cells/mℓ and cultured at 37°C for 3 days in a spinner culture bottle.

Then, the cells were collected from a portion (250 mℓ) of the cell suspension by centrifugation under 1,100 x G at 4°C for 10 minutes, washed with 80 mℓ of phosphate buffer (pH 7.0), and solubilized in 10 mℓ of a solution comprising 5 M guanidium thiocyanate, 10 mM EDTA, 50 mM Tris-HCl (pH 7) and 8% (V/V) 2-mercaptoethanol by means of a Voltex mixer. The solubilized product was transferred into a centrifuge tube, and 80 mℓ of a 4 M LiCl solution was added. The mixture was stirred and then allowed to stand at 4°C for 20 hours.

After centrifugation in Hitachi RPR10 rotor (made by Hitachi, Ltd.) at 9,000 rpm for 90 minutes, RNA was recovered as a precipitate. The RNA precipitate was suspended in 50 mℓ of a solution comprising 4 M urea and 2 M lithium chloride, and the suspension was centrifuged in Hitachi RPR10 rotor at 9,000 rpm for 60 minutes, whereby RNA was again recovered as a precipitate. The RNA precipitate was dissolved in 10 mℓ of a solution comprising 0.1% sodium laurylsulfate, 1 mM EDTA and 10 mM Tris-HCl (pH 7.5), extracted with phenol and chloroform and recovered by ethanol precipitation. About 2.5 mg of the thus obtained RNA was dissolved in 1 mℓ of a solution comprising 10 mM Tris-HCl (pH 8.0) and 1 mM EDTA, incubated at 65°C for 5 minutes, and admixed with 0.1 mℓ of 5 M NaCl. The mixture was subjected to chromatography with an oligo(dT) cellulose column (made by P-L Biochemicals) (column volume: 0.5 mℓ). The adsorbed mRNA having poly(A) was eluted with a solution comprising 10 mM Tris-HCl (pH 7.5) and 1 mM EDTA, whereby about 100 µg of the mRNA having poly(A) was obtained.

Example 2
Synthesis of a cDNA and insertion of the cDNA into a vector:

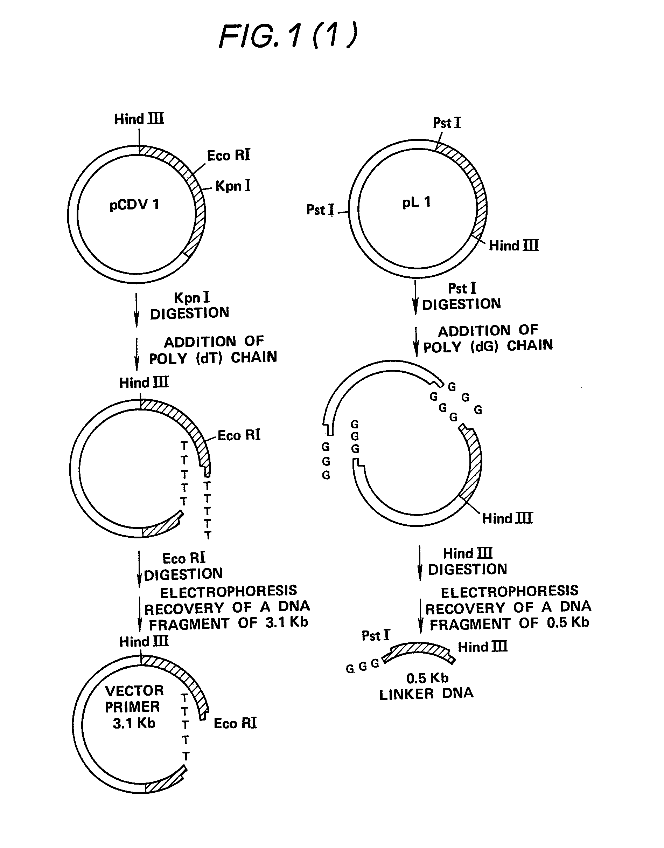

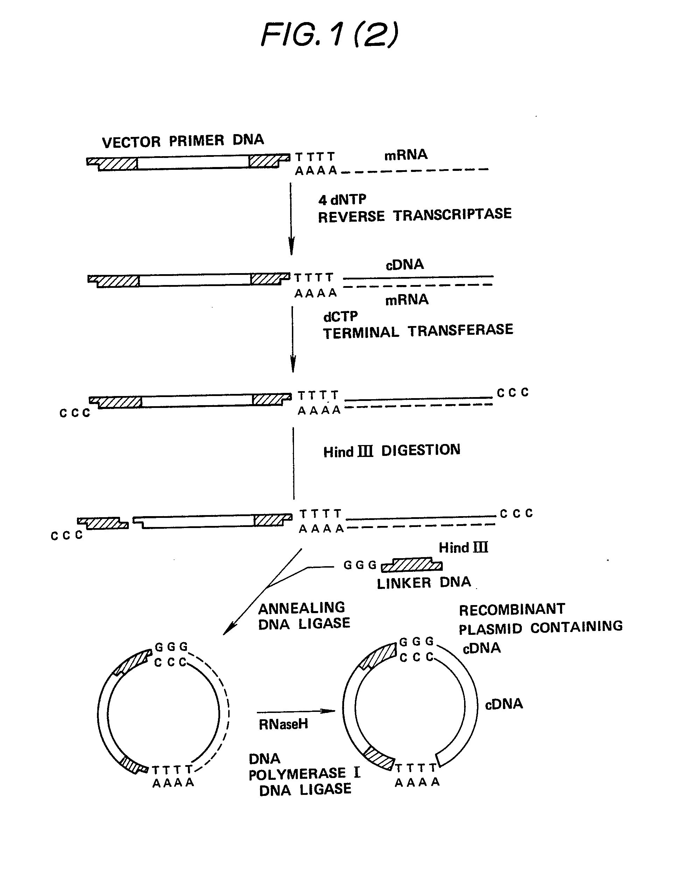

Synthesis of a cDNA and construction of a recombinant plasmid carrying the cDNA were carried out according to the method of Okayama-Berg [Mol. Cell. Biol. 2, 161 (1982)]. The process is outlined in Fig. 1.

First, 400 µg of pcDV1 (made by Pharmacia Fine Chemicals Inc.) [Okayama & Berg: Mol. Cell. Biol. 3, 280 (1983)] was added to 300 µℓ of a solution comprising 10 mM Tris-HCl (pH 7.5), 6 mM $MgCl_2$ and 10 mM NaCl, and 500 units of KpnI was further added thereto. The mixture was subjected to reaction at 37°C for 6 hours to cleave the plasmid at the KpnI site. After extraction with phenol-chloroform, DNA was recovered by ethanol precipitation. Then, about 200 µg of the DNA cleaved with KpnI was added to 200 µℓ of a solution prepared by adding 0.25 mM dTTP to a buffer comprising 40 mM sodium cacodylate, 30 mM Tris-HCl (pH 6.8), 1 mM $CaCl_2$ and 0.1 mM dithiothreitol (hereinafter abbreviated as DTT) (the buffer is hereinafter referred to as TdT buffer), and further 81 units of terminal deoxynucleotidyl transferase (made by P-L Biochemicals, hereinafter abbreviated as TdT) was added thereto. The mixture was subjected to reaction at 37°C for 11 minutes, whereby poly(dT) chains (about 67 mer) were added to the 3'-ends of pcDV1 cleaved with KpnI. About 100 µg of pcDV1 DNA with the poly(dT) chains was recovered from the solution by phenol-chloroform extraction and ethanol precipitation. The thus obtained DNA was added to 150 µℓ of a buffer comprising 10 mM Tris-HCl (pH 7.5), 6 mM $MgCl_2$ and 100 mM

NaCl, and further, 360 units of EcoRI was added thereto. The mixture was subjected to reaction at 37°C for 2 hours. Then, the reaction product was treated according to LGT method to obtain DNA fragments of about 3.1 Kb which is about 60 μg of pcDV1 with poly(dT) chains. The DNA was dissolved in 500 μℓ of a solution comprising 10 mM Tris-HCl (pH 8.0) and 1 mM EDTA, and incubated at 65°C for 5 minutes. Then, the mixture was cooled with ice and 50 μℓ of 5 M NaCl was added thereto. The mixture was subjected to chromatography with an oligo(dA) cellulose column (made by Collaborative Research). The DNAs with sufficiently long poly(dT) chains were adsorbed on the column, and eluted with a solution comprising 10 mM Tris-HCl (pH 8.0) and 1 mM EDTA, whereby 27 μg of pcDV1 with poly(dT) chains (hereinafter referred to as vector primer) was obtained.

Then, a linker DNA was prepared.

First, about 14 μg of pL1 (made by Pharmacia Fine Chemicals Inc.) [Okayama & Berg: Mol. Cell. Biol., 3, 280 (1983)] was added to 200 μℓ of a buffer comprising 10 mM Tris-HCl (pH 7.5), 6 mM MgCl$_2$ and 50 mM NaCl, and further, 50 units of PstI was added thereto. The mixture was subjected to reaction at 37°C for 4 hours to cleave pL1 DNA at the PstI site. The reaction product was subjected to phenol-chloroform extraction and ethanol precipitation, whereby about 13 μg of pL1 DNA cleaved with PstI was recovered. About 13 μg of the DNA was added to 50 μℓ of TdT buffer containing 0.25 mM (final concentration) dGTP, and further, 54 units of TdT (made by P-L Biochemicals) was added thereto. The mixture was incubated at 37°C for 13 minutes to add (dG) chains (about 14 mer) to the 3'-ends of pL1 cleaved with PstI. After extraction with phenol-chloroform, DNA was recovered by ethanol precipitation. Then, the DNA was added to 100 μℓ of a buffer comprising 10 mM Tris-HCl (pH 7.5), 6 mM MgCl$_2$ and 60 mM NaCl, and further, 80 units of HindIII was added thereto. The mixture was incubated at 37°C for 3 hours to cleave pL1 DNA at the HindIII site.

The reaction product was fractionated by agarose gel electrophoresis, and DNA fragments of about 0.5 Kb were recovered by DEAE paper method [Dretzen, et al.: Anal. Biochem., 112, 295 (1981)], whereby linker DNA with oligo(dG) chains (hereinafter referred to as linker DNA) was obtained.

About 2 µg of the poly(A)RNA and about 1.4 µg of the vector primer prepared above were dissolved in 22.3 µℓ of a solution comprising 50 mM Tris-HCl (pH 8.3), 8 mM MgCl$_2$, 30 mM KCl, 0.3 mM DTT, 2 mM dNTP (dATP, dTTP, dGTP and dCTP) and 10 units of ribonuclease inhibitor (made by P-L Biochemicals), and further, 10 units of reverse transcriptase (made by Seikagaku Kogyo) was added thereto. The mixture was incubated at 41°C for 90 minutes to synthesize a DNA complementary to the mRNA. The reaction product was subjected to phenol-chloroform extraction and ethanol precipitation to recover a vector primer DNA with RNA-DNA double stranded chain. The DNA was dissolved in 20 µℓ of TdT buffer containing 66 µM dCTP and 0.2 µg of poly(A), and further, 14 units of TdT (made by P-L Biochemicals) was added thereto. The mixture was incubated at 37°C for 2 minutes to add (dC) chains (20 mer) to the 3'-ends of the cDNA. The reaction product was extracted with phenol-chloroform, and a cDNA-vector primer DNA with (dC) chains was recovered by ethanol precipitation. The DNA was dissolved in 400 µℓ of a solution comprising 10 mM Tris-HCl (pH 7.5), 6 mM MgCl$_2$ and 60 mM NaCl, and further, 20 units of HindIII was added thereto. The mixture was incubated at 37°C for 2 hours to cleave the DNA at the HindIII site. The reaction product was subjected to phenol-chloroform extraction and ethanol precipitation, whereby 0.5 picomole of a cDNA-vector primer DNA with (dC) chains was obtained. Then, 0.2 picomole of the DNA and 0.4 picomole of the said linker DNA were dissolved in 100 µℓ of a solution comprising 10 mM Tris-HCl (pH 7.5), 0.1 M NaCl and 1 mM EDTA, and incubated at 65°C, 42°C and 0°C for 10 minutes, 25 minutes and 30 minutes, respectively. The reaction solution was adjusted to 1000 µℓ (total volume) of a solution having a composition of 20 mM Tris-HCl (pH 7.5), 4 mM MgCl$_2$, 10 mM (NH$_4$)$_2$SO$_4$, 0.1 M KCl and 0.1 mM β-NAD. Then, 25 units of Escherichia coli DNA ligase (made by New England Biolabs) was added to the reaction solution, and incubation was carried out at 11°C for 18 hours. The reaction solution was further adjusted to a solution containing 40 μM each dNTP and 0.15 mM β-NAD, and then 10 units of Escherichia coli DNA ligase, 20 units of Escherichia coli DNA polymerase I (made by P-L Biochemicals) and 10 units of Escherichia coli ribonuclease H (made by P-L Biochemicals) were added. The mixture was incubated successively at 12°C and 25°C each for one hour. Cyclization of a recombinant DNA containing the cDNA and replacement of the RNA moiety of the RNA-DNA double stranded chain with the DNA were carried out by the said reaction to form a recombinant plasmid having complete double stranded DNA.

Example 3

Selection of a recombinant DNA containing physiologically active polypeptide cDNA:

Escherichia coli C600 SF8 strain [Cameron, et al.: Proc. Natl. Acad. Sci., USA, 72, 3416 (1975); deposited with the FRI on May 27, 1986 as FERM BP-1070] was subjected to transformation using the recombinant plasmid obtained in Example 2 according to the method of Scott, et al. [Katsuya Shigesada: Saibo Kogaku (Cell Engineering), 2, 616 (1983)]. About 10,000 colonies thus obtained were immobilized on nitrocellulose filters. A synthetic DNA corresponding to the amino acid sequence from the first to 15th amino acids from the N-terminal of the ubiquitin precursor of the human ubiquitin precursor gene isolated by Wiborg, et al. [O. Wiborg, et al.: EMBO J. 4, 755 (1985)], that is,

5′-A T G C A G A T C T T C G T G A A G A C C C T

G A C T G G T A A G A C C A T C A C C C T C-3′

and a synthetic DNA corresponding to the amino acid sequence from the 30th to 35th amino acids from the N-terminal, that is,

5′-A T C C A A G A T A A G G A A G G-3′
   (T)   (G)   (C)   (A)   (G)
   (A)

(the third base is C, T or A; the 6th is A or G, the 9th is T or C, the 12th is G or A; the 15th is A or G, and combination of the bases makes 48 kinds of synthetic DNAs) were labelled with $^{32}p$ and used as probes. Eight strains which strongly hybridized with the former probe at 45°C and with the latter probe at 40°C were selected according to the method of Grunstein-Hogness [Proc. Natl. Acad. Sci., USA, 72, 3961 (1975)].

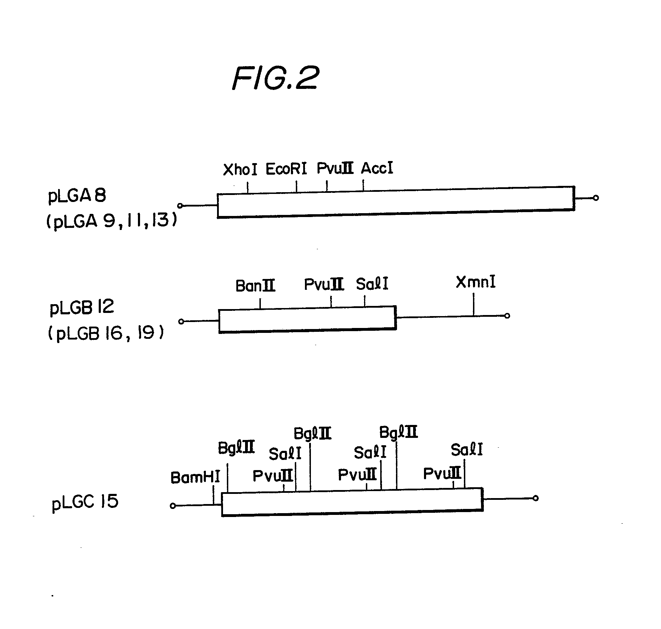

These plasmids were named pLGA8, pLGA9, pLGA11, pLGA13, pLGB12, pLGB16, pLGB19 and pLGC15, respectively. The thus obtained 8 kinds of plasmids were digested with various restriction enzymes to determine cleavage maps of the cDNA moiety. From the positions of the restriction enzyme sites, the obtained plasmids could be classified into three groups, i.e. a group of pLGA8, pLGA9, pLGA11 and pLGA13, a group of pLGB12, pLGB16 and pLGB19, and pLGC15. The restriction enzyme maps of the three groups are shown in Fig. 2.

Then, plasmids pLGA8, pLGB12 and pLGC15 which seemed to contain a cDNA with almost complete length were selected from the respective groups and introduced into animal cells for expression. As a host, COS-1 cell strain ATCC CRL 1650 was used. Since the COS-1 cell strain constitutively expresses T-antigen of SV40 virus, transient expression of pLGA8, pLGB12 and PLGC15 having the origin of replication of the virus and such a structure that a gene is inserted downstream from an early gene promoter of the virus is possible.  Introduction of pLGA8, pLGB12 and pLGC15 into the COS-1 cell strain was carried out according to the DEAE-dextran method.  That is, the cells were inoculated into 45 mℓ of Dulbecco's Modified Eagle (DME) medium (made by Nissui Seiyaku) containing 10% fetal calf serum (FCS) in $2 \times 10^5$ cells/mℓ [three 75 mℓ-tissue culture flasks made by Corning Glass Works were used for culturing (15 mℓ/flask)] and cultured at 37°C for 3 days in a $CO_2$ incubator.  Then, 21 mℓ of DME medium containing 50 mM Tris-HCl (pH 7.3) in which 150 µg of pLGA8, pLGB12 or pLGC15 was dissolved and 500 µg/mℓ DEAE-dextran was added to the thus cultured COS-1 cell strain, and the mixture was incubated for 8 hours.  The cells were washed and mixed with 30 mℓ of DME medium containing 10% fetal calf serum (FCS).  The mixture was further incubated for 3 days.  Then, the cells were washed and suspended in 2 mℓ of PBS [8 g/ℓ NaCl, 0.2 g/ℓ KCl, 1.15 g/ℓ $Na_2HPO_4$ (anhydrous) and 0.2 g/ℓ $KH_2PO_4$], followed by disruption in a Teflon homogenizer.  The supernatant obtained by centrifugation at 12,000 rpm for 20 minutes was dialyzed against PBS at 4°C.

Cell growth-promoting activity was determined with human lymphoblastoid cell strain Namalwa (ATCC CRL 1432).  That is, 5 to $10 \times 10^5$ cells/mℓ Namalwa strain was cultured at 37°C for 48 hours in 75 mℓ of RPMI-1640 medium (made by Nissui Seiyaku) further containing glutamine (4 mM), streptomycin (25 µg/mℓ), penicillin (25 U/mℓ), HEPES (10 mM), sodium bicarbonate (0.01%) and fetal calf serum (10%).  The cells were collected by centrifuging the culture broth under 800 x G for 5 minutes, washed with a medium having the same composition as mentioned above except that serum is not contained, and poured into a 24-well multidish plate (made by Corning Glass Works).  Then, 0.9 mℓ of the serum-free medium and 0.1 mℓ of the sample solution were added thereto. The mixture was cultured at 37°C for 3 days in a $CO_2$ incubator, and the number of cells was counted with a hemacytometer. The results are shown in Table 1. As the cell growth-promoting activity upon Namarwa strain was apparently observed, it has been confirmed that pLGA8, pLGB12 and pLGC15 coded for the physiologically active polypeptide.

Escherichia coli strain containing pLGA8 was deposited with the FRI on May 27, 1986 as Escherichia coli ELGA8 FERM BP-1067.

Table 1

| Plasmid | Propagation rate (2) |
| --- | --- |
| pLGA8 | 2.29 |
| pLGB12 | 1.55 |
| pLGC15 | 1.45 |
| free of DNA (1) | 1.00 |

(1)   A cell extract solution was prepared from COS-1 cells subjected to the same operations as above without addition of DNA, and assayed.

(2)   Propagation rate is relative to the propagation obtained in (1) as 1.00.

The complete nucleotide sequences of the coding region of the cDNAs of pLGA8, pLGB12 and pLGC15 were determined according to the dideoxy sequence method [J. Messing, et al.: Gene 19, 269 (1985)] using M13 phage.

The amino acid sequences expected from the sequences of the cDNAs of pLGA8, pLGB12 and pLGC15 are respectively shown in Tables 2, 3 and 6. It was found that they had an amino acid sequence corresponding to that of human ubiquitin as a part of the structure.

Table 2

```
          10        20        30        40        50        60
ATGCAGATTTTCGTGAAAACCCTTACGGGGAAGACCATCACCCTCGAGGTTGAACCCTCG
MetGlnIlePheValLysThrLeuThrGlyLysThrIleThrLeuGluValGluProSer

          70        80        90       100       110       120
GATACGATAGAAAATGTAAAGGCCAAGATCCAGGATAAGGAAGGAATTCCTCCTGATCAG
AspThrIleGluAsnValLysAlaLysIleGlnAspLysGluGlyIleProProAspGln

         130       140       150       160       170       180
CAGAGACTGATCTTTGCTGGCAAGCAGCTGGAAGATGGACGTACTTTGTCTGACTACAAT
GlnArgLeuIlePheAlaGlyLysGlnLeuGluAspGlyArgThrLeuSerAspTyrAsn

         190       200       210       220       230       240
ATTCAAAAGGAGTCTACTCTTCATCTTGTGTTGAGACTTCGTGGTGGTGCTAAGAAAAGG
IleGlnLysGluSerThrLeuHisLeuValLeuArgLeuArgGlyGlyAlaLysLysArg

         250       260       270       280       290       300
AAGAAGAAGTCTTACACCACTCCCAAGAAGAATAAGCACAAGAGAAAGAAGGTTAAGCTG
LysLysLysSerTyrThrThrProLysLysAsnLysHisLysArgLysLysValLysLeu

         310       320       330       340       350       360
GCTGTCCTGAAATATTATAAGGTGGATGAGAATGGCAAAATTAGTCGCCTTCGTCGAGAG
AlaValLeuLysTyrTyrLysValAspGluAsnGlyLysIleSerArgLeuArgArgGlu

         370       380       390       400       410       420
TGCCCTTCTGATGAATGTGGTGCTGGGGTGTTTATGGCAAGTCACTTTGACAGACATTAT
CysProSerAspGluCysGlyAlaGlyValPheMetAlaSerHisPheAspArgHisTyr

         430       440       450       460       470
TGTGGCAAATGTTGTCTGACTTACTGTTTCAACAAACCAGAAGACAAGTAA
CysGlyLysCysCysLeuThrTyrCysPheAsnLysProGluAspLys***
```

Table 3

```
           10        20        30        40        50        60
ATGCAAATATTCGTGAAGACCCTGACCGGCAAGACCATCACTCTGGAGGTGGAGCCCAGT
MetGlnIlePheValLysThrLeuThrGlyLysThrIleThrLeuGluValGluProSer

           70        80        90       100       110       120
GACACCATCGAAAATGTGAAGGCCAAGATCCAAGATAAAGAAGGCATCCCCCCCGACCAG
AspThrIleGluAsnValLysAlaLysIleGlnAspLysGluGlyIleProProAspGln

          130       140       150       160       170       180
CAGAGGCTCATCTTTGCAGGCAAGCAGCTGGAAGATGGCCGCACTCTTTCTGACTACAAC
GlnArgLeuIlePheAlaGlyLysGlnLeuGluAspGlyArgThrLeuSerAspTyrAsn

          190       200       210       220       230
ATCCAGAAAGAGTCGACCCTGCACCTGGTCCTGCGCCTGAGGGGTGGCTGTTAA
IleGlnLysGluSerThrLeuHisLeuValLeuArgLeuArgGlyGlyCys***
```

Table 6

```
           10        20        30        40        50        60
ATGCAGATCTTCGTGAAAACCCTTACCGGCAAGACCATCACCCTTGAGGTGGAGCCCAGT
MetGlnIlePheValLysThrLeuThrGlyLysThrIleThrLeuGluValGluProSer

           70        80        90       100       110       120
GACACCATCGAAAATGTGAAGGCCAAGATCCAGGATAAGGAAGGCATTCCCCCCGACCAG
AspThrIleGluAsnValLysAlaLysIleGlnAspLysGluGlyIleProProAspGln

          130       140       150       160       170       180
CAGAGGCTCATCTTTGCAGGCAAGCAGCTGGAAGATGGCCGTACTCTTTCTGACTACAAC
GlnArgLeuIlePheAlaGlyLysGlnLeuGluAspGlyArgThrLeuSerAspTyrAsn

          190       200       210       220       230       240
ATCCAGAAGGAGTCGACCCTGCACCTGGTCCTGCGTCTGAGAGGTGGTATGCAGATCTTC
IleGlnLysGluSerThrLeuHisLeuValLeuArgLeuArgGlyGlyMetGlnIlePhe

          250       260       270       280       290       300
GTGAAGACCCTGACCGGCAAGACCATCACCCTGGAAGTGGAGCCCAGTGACACCATCGAA
ValLysThrLeuThrGlyLysThrIleThrLeuGluValGluProSerAspThrIleGlu
```

```
           310       320       330       340       350       360
AATGTGAAGGCCAAGATCCAGGATAAAGAAGGCATCCCTCCCGACCAGCAGAGGCTCATC
AsnValLysAlaLysIleGlnAspLysGluGlyIleProProAspGlnGlnArgLeuIle

           370     . 380       390       400       410       420
TTTGCAGGCAAGCAGCTGGAAGATGGCCGCACTCTTTCTGACTACAACATCCAGAAGGAG
PheAlaGlyLysGlnLeuGluAspGlyArgThrLeuSerAspTyrAsnIleGlnLysGlu

           430       440       450       460       470       480
TCGACCCTGCACCTGGTCCTGCGTCTGAGAGGTGGTATGCAGATCTTCGTGAAGACCCTG
SerThrLeuHisLeuValLeuArgLeuArgGlyGlyMetGlnIlePheValLysThrLeu

           490       500       510       520       530       540
ACCGGCAAGACCATCACTCTGGAGGTGGAGCCCAGTGACACCATCGAAAATGTGAAGGCC
ThrGlyLysThrIleThrLeuGluValGluProSerAspThrIleGluAsnValLysAla

           550       560       570       580      .590     ·  600
AAGATCCAAGATAAAGAAGGCATCCCCCCCGACCAGCAGAGGCTCATCTTTGCAGGCAAG
LysIleGlnAspLysGluGlyIleProProAspGlnGlnArgLeuIlePheAlaGlyLys

           610       620       630      .640       650       660
CAGCTGGAAGATGGCCGCACTCTTTCTGACTACAACATCCAGAAAGAGTCGACCCTGCAC
GlnLeuGluAspGlyArgThrLeuSerAspTyrAsnIleGlnLysGluSerThrLeuHis

           670       680       690
CTGGTCCTGCGCCTGAGGGGTGGCTGTTAA
LeuValLeuArgLeuArgGlyGlyCys***
```

Example 4

Construction of recombinant plasmid pLGB-al:

Escherichia coli C600 SF8 strain carrying pLGB12 (3.6 Kb) obtained in Examples 1, 2 and 3 was cultured, and pLGB12 DNA was prepared from the cultured cells according to the conventional process. Then, 3 µg of the thus obtained pLGB12 DNA was dissolved in 40 µℓ (total volume) of a solution containing 10 mM Tris-HCl (pH 7.5), 7 mM $MgCl_2$ and 6 mM 2-mercaptoethanol (the solution is hereinafter referred to as "Y-0 buffer"), and 6 units of restriction enzyme HpaII (made by New England Biolabs) was added thereto. The mixture was subjected to cleavage reaction at 37°C for 2 hours. NaCl was added thereto to make the ultimate concentration of 100 mM, and 6 units of restriction enzyme BamHI (made by Takara Shuzo; the restriction enzymes used hereinafter are all products of Takara Shuzo, unless otherwise specified) was added thereto. The mixture was subjected to cleavage reaction at 37°C for 2 hours. About 0.2 µg of DNA fragments (HpaII-BamHI fragments) of about 490 bp containing a large portion of the physiologically active polypeptide was obtained from the reaction solution by LGT method.

Separately, 2 µg of pKYP10 (4.7 Kb) prepared according to the process disclosed in Japanese Published Unexamined Patent Application No. 110600/83 was dissolved in 40 µℓ (total volume) of a solution containing 10 mM Tris-HCl (pH 7.5), 7 mM MgCl$_2$, 6 mM 2-mercaptoethanol and 100 mM NaCl (the solution is hereinafter referred to as "Y-100 buffer"), and 6 units each of restriction enzymes HindIII and BamHI were added thereto. The mixture was subjected to cleavage reaction at 37°C for 2 hours, and about 1.5 µg of DNA fragments (BamHI-HindIII fragments) of about 4.4 Kb containing a tryptophan promoter (Ptrp) was obtained from the reaction solution by LGT method.

The following DNA linker was synthesized in order to furnish the sequence from Met (ATG) at the N-terminal of the physiologically active polypeptide to the second base (AC) of Thr (ACC) as the 9th amino acid thereof and to adjust the distance between the SD sequence downstream from Ptrp and the ATG to an appropriate length of 6 to 18 bp.

```
     HindIII              31-mer              HpaII
       |      Met Gln Ile Phe Val Lys Thr Leu  |
    5'-|AGCTT ATG CAA ATA TTC GTA AAG ACG TTA AC|  -3'
    3'-  |A TAC GTT TAT AAG CAT TTC TGC AAT TGGC|  -5'
                             29-mer
```

First, two single stranded DNAs of 31-mer and 29-mer were synthesized by the ordinary triester method [R. Crea, et al.: Proc. Natl. Acad. Sci., USA 75, 5765 (1978)]. Then, 20 picomoles each of the 31-mer and 29-mer DNAs were dissolved in 40 μℓ (total volume) of a solution containing 50 mM Tris-HCl (pH 7.5), 10 mM MgCl2, 5 mM dithiothreitol, 0.1 mM EDTA and 1 mM ATP, and one unit of T4 polynucleotide kinase (made by Takara Shuzo) was added thereto. The mixture was subjected to phosphorylation reaction at 37°C for 60 minutes.

Then, 0.1 μg of the HpaII-BamHI fragments (about 490 bp) derived from pLGB12 and 0.5 μg of the BamHI-HindIII fragments (about 4.4 Kb) of expression vector pKYP10, which were obtained above, were dissolved in 20 μℓ (total volume) of a buffer containing 20 mM Tris-HCl (pH 7.6), 10 mM MgCl2, 10 mM dithiothreitol and 1 mM ATP (the buffer is hereinafter referred to as "T4 ligase buffer"), and about one picomole of the said DNA linker was added to the mixture. Furthermore, 50 units of T4 DNA ligase (made by Takara Shuzo: the same shall apply hereinafter) was added to the mixture. The mixture was subjected to reaction at 4°C for 18 hours.

Escherichia coli C600 SF8 strain was transformed with the reaction mixture containing the recombinant plasmid to obtain Ap^r colonies. Plasmid DNA was separated and purified from the transformant by the known method [H.C. Birnboim, et al.: Nucleic Acids Res. 7, 1513 (1979)] (this method is used hereinafter for preparing plasmid DNA). The structure of the thus obtained plasmid was confirmed by agarose gel electrophoresis after cleavage with restriction enzymes PvuII and BamHI. The plasmid was named pLGB-al. It was confirmed by the dideoxy sequence method that the nucleotide sequence around the HindIII site of pLGB-al was as follows:

```
HindIII
   |           Met Gln Ile Phe Val Lys Thr Leu Thr Gly Lys
   |AGCT  T ATG CAA ATA TTC GTA AAG ACG TTA ACC GGC AAG
   ↓
```

Example 5

Construction of recombinant plasmid pLGB-d2:

Escherichia coli C600 SF8 strain carrying pLGB-al (4.9 Kb) obtained in Example 4 was cultured, and pLGB-al DNA was prepared from the cultured cells according to the ordinary process. Then, 5 µg of the thus obtained pLGB-al DNA was dissolved in 80 µℓ of Y-100 buffer, and 10 units each of restriction enzymes BanIII (made by Toyobo) and BamHI were added thereto. The mixture was subjected to cleavage reaction at 37°C for 2 hours, and about 0.3 µg of DNA fragments (BanIII-BamHI fragments) of about 530 bp containing the said physiologically active polypeptide gene was obtained from the reaction solution by LGT method.

Separately, 2 µg of pGEL1 (3.4 Kb) was dissolved in 40 µℓ of Y-100 buffer, and 4 units each of BanIII and BamHI were added thereto. The mixture was subjected to cleavage reaction at 37°C for 2 hours, and about 1 µg of DNA fragments (BanIII-BamHI fragments) of about 2.7 Kb containing a tryptophan tandem promoter (Ptrp x 2) was obtained from the reaction solution by LGT method.

Then, 0.2 µg of the BanIII-BamHI fragments (about 530 bp) derived from pLGB-al and 0.5 µg of the BanIII-BamHI fragments (about 2.7 Kb) derived from pGEL1, which were obtained above, were dissolved in 20 µℓ (total volume) of T4 ligase buffer, and 2 units of T4 DNA ligase was added thereto. The mixture was subjected to ligation reaction at 4°C for 18 hours.

Escherichia coli C600 SF8 strain was transformed with the thus obtained mixture of recombinant plasmids to obtain Ap[r] colonies. Plasmid DNA was recovered from the cultured cells of the colonies to obtain pLGB-d2. The structure of pLGB-d2 was confirmed by agarose gel electrophoresis after cleavage with restriction enzymes EcoRI, PvuII, BamHI and PstI.

Example 6

Construction of recombinant plasmid pLGA-d5:

Escherichia coli C600 SF8 strain carrying pLGB-d2 (3.2 Kb) obtained in Example 5 was cultured, and pLGB-d2 DNA was prepared from the cultured cells according to the ordinary process. Then, 2 μg of the thus obtained pLGB-d2 DNA was dissolved in 40 μℓ of Y-100 buffer, and 4 units each of PvuII and BamHI were added thereto. The mixture was subjected to cleavage reaction at 37°C for 2 hours, and about 1 μg of DNA fragments (PvuII-BamHI fragments) of about 2.8 Kb containing Ptrp x 2 was obtained from the reaction solution by LGT method.

Separately, Escherichia coli C600 SF8 strain carrying pLGA8 (3.7 Kb) obtained in Examples 1, 2 and 3 was cultured, and pLGA8 DNA was prepared from the cultured cells according to the ordinary process. Then, 3 μg of the thus obtained pLGA8 DNA was dissolved in 40 μℓ of Y-100 buffer, and 6 units each of PvuII and BamHI were added thereto. The mixture was subjected to cleavage reaction at 37°C for 2 hours, and about 0.2 μg of DNA fragments (PvuII-BamHI fragments) of about 400 bp containing the DNA coding for C-terminal region of the physiologically active polypeptide was obtained from the reaction solution by LGT method.

Then, 0.3 μg of the PvuII-BamHI fragments (about 2.8 Kb) derived from pLGB-d2 and 0.1 μg of the PvuII-BamHI fragments (about 400 bp) derived from pLGA8, which were obtained above, were dissolved in 20 μℓ (total volume) of T4 ligase buffer, and 20 units of T4 DNA ligase was added thereto. The mixture was subjected to ligation reaction at 4°C for 18 hours.

Escherichia coli C600 SF8 strain was transformed with the thus obtained mixture of recombinant plasmids to obtain Ap$^r$ colonies. Plasmid DNA was recovered from the cultured cells of the colonies to obtain pLGA-d5. The structure of pLGA-d5 was confirmed by agarose gel electrophoresis after cleavage with restriction enzymes PvuII, BamHI, EcoRI and PstI.

Example 7

Construction of recombinant plasmid pLGA-el:

Escherichia coli C600 SF8 strain carrying pLGA-d5 (3.2 Kb) obtained in Example 6 was cultured, and pLGA-d5 DNA was prepared from the cultured cells according to the ordinary process. Then, 2 µg of the thus obtained pLGA-d5 DNA was dissolved in 40 µℓ of Y-100 buffer, and 4 units each of BanIII and PstI were added thereto. The mixture was subjected to cleavage reaction at 37°C for 2 hours, and about 1 µg of DNA fragments (BanIII-PstI fragments) of about 2.2 Kb containing the physiologically active polypeptide gene was obtained from the reaction solution by LGT method.

Separately, 4 µg of pKYP10 (4.7 Kb) prepared according to the process disclosed in Japanese Published Unexamined Patent Application No. 110600/83 was dissolved in 80 µℓ of Y-100 buffer, and 8 units each of BanIII and PstI were added thereto. The mixture was subjected to cleavage reaction at 37°C for 2 hours, and about 0.5 µg of DNA fragments (BanIII-PstI fragments) of about 1.1 Kb containing Ptrp was obtained from the reaction solution by LGT method.

Then, 0.5 µg of the BanIII-PstI fragments (about 2.2 Kb) derived from pLGA-d5 and 0.2 µg of the BanIII-PstI fragments derived from pKYP10, which were obtained above, were dissolved in 20 µℓ (total volume) of T4 ligase buffer, and 2 units of T4 DNA ligase was added thereto. The mixture was subjected to ligation reaction at 4°C for 18 hours.

Escherichia coli C600 SF8 strain was transformed with the thus obtained mixture of recombinant plasmids to obtain Ap$^r$ colonies. Plasmid DNA was recovered from the cultured cells of the colonies to obtain pLGA-el. The structure of pLGA-el was confirmed by agarose gel electrophoresis after cleavage with restriction enzymes PvuII, PstI and BamHI.

Example 8

Production of the physiologically active polypeptide by Escherichia coli strains carrying pLGB-al and pLGA-el:

Escherichia coli C600 SF8 strains respectively carrying recombinant plasmids pLGB-al and pLGA-el obtained in Examples 4 and 7 [C600 SF8 strain carrying pLGB-al was named ELGB-al and deposited with the FRI on May 27, 1986 as FERM BP-1068] were cultured at 37°C for 18 hours in LG medium prepared by dissolving 10 g of Bacto-Tryptone, 5 g of yeast extract, 5 g of NaCl and 1 g of glucose in 1 ℓ of water and adjusting the pH to 7.0 with NaOH. Then, 0.5 mℓ of the culture liquor was inoculated into 10 mℓ of MCG medium (0.6% Na$_2$HPO$_4$, 0.3% KH$_2$PO$_4$, 0.5% NaCl, 0.5% Casamino acids, 1 mM MgSO$_4$, and 4 µg/mℓ vitamin B1; pH 7.2) containing 25 µg/mℓ tryptophan and 50 µg/mℓ ampicillin. After culturing at 30°C for 4 to 8 hours, 3β-indoleacrylic acid (hereinafter abbreviated as IAA) which is a tryptophan-operon-inducing substance was added thereto to make a concentration of 10 µg/mℓ, and culturing was continued for further 2 to 12 hours. Then, the culture liquor was centrifuged at 8,000 rpm for 10 minutes to collect the cells. The cells were washed with PBS, and the washed cells were suspended in 5 mℓ of PBS and subjected to ultrasonic disruption at 0°C for 10 minutes with Sonifier cell disruptor 200 (Branson Sonic Power Company, output control 2). By centrifugation at 15,000 rpm for 30 minutes, the physiologically active polypeptide was separated and extracted into the supernatant. After the cells were removed with a 0.45 µm filter (made by Nihon Millipore, Limited), the cell growth-promoting activity was measured according to the process disclosed in Example 3. The results of the measurement are shown in Table 4.

Table 4

| Plasmid | Propagation rate (2) |
|---------|----------------------|
| pLGB-al | 1.41 |
| pLGA-el | 2.43 |
| pcDVl (1) | 1.00 |

(1) A cell extract solution was prepared from C600 SF8 strain carrying plasmid (pcDVl) which does not contain the DNA coding for the physiologically active polypeptide in the same manner as described above, and assayed.

(2) Propagation rate is relative to the propagation obtained in (1) as 1.00.

Example 9

Construction of recombinant plasmid pLGC:Scll:

Escherichia coli C600 SF8 strain carrying pLGC15 (4.2 Kb) obtained in Example 3 was cultured, and pLGC15 DNA was prepared from the cultured cells by a conventional method. 3 µg of the thus obtained pLGC15 DNA was dissolved in 60 µℓ (total volume) of Y-100 buffer, and 6 units of restriction enzyme XhoI was added thereto. Cleavage reaction was carried out at 37°C for 2 hours, and about 0.5 µg of DNA fragments (XhoI fragments) of about 1.3 Kb containing the physiologically active polypeptide (UL-C) gene was obtained from the reaction solution by LGT method.

Separately, 2 µg of pUCl9 (2.7 Kb) was dissolved in 40 µℓ (total volume) of a solution containing 10 mM Tris-HCl

(pH 7.5), 7 mM MgCl$_2$, 6 mM 2-mercaptoethanol and 175 mM NaCl, and 10 units of restriction enzyme SalI was added thereto. Cleavage reaction was carried out at 37°C for 2 hours, and about 1.0 μg of DNA fragments (SalI fragments) of about 2.7 Kb was obtained from the reaction solution by LGT method. Then, 0.4 μg of the XhoI fragments (about 1.3 Kb) obtained from pLGC15 as above and 0.3 μg of the SalI fragments (about 2.7 Kb) obtained from pUC19 as above were dissolved in 20 μℓ (total volume) of T4 ligase buffer. 2 units of T4 DNA ligase was added and reaction was carried out at 4°C for 18 hours.

Escherichia coli C600 SF8 strain was transformed using the reaction mixture containing recombinant plasmids to obtain Ap$^r$ colonies. Plasmid DNA was isolated and purified from the transformants by the method of Birnboim, et al. The structure of the thus obtained plasmid was confirmed by polyacrylamide gel electrophoresis after cleavage with SalI and BamHI. The plasmid was named pLGC:Scll.

Example 10

Construction of recombinant plasmid pLGC-x1:

Escherichia coli C600 SF8 strain carrying pLGC:Scll (4.0 Kb) obtained in Example 9 was cultured, and pLGC:Scll DNA was prepared from the cultured cells by a conventional method. 10 μg of the obtained pLGC:Scll DNA was dissolved in 100 μℓ of Y-100 buffer, and 10 units of restriction enzyme BglII was added thereto. Cleavage reaction was carried out at 37°C for one hour, whereby pLGC:Scll DNA was partially cleaved with BglII. After phenol extraction and chloroform extraction, about 8.0 μg of DNA fragments were obtained by ethanol precipitation. Then, 8.0 μg of the DNA fragments of 4.0 Kb was dissolved in a solution containing 50 mM Tris-HCl (pH 7.8), 7 mM MgCl$_2$ and 6 mM 2-mercaptoethanol, and 1 mM each dATP, dTTP, dCTP and dGTP were added thereto (total volume: 160 μℓ). Further, 5 units of Klenow fragment (product of Takara Shuzo) was added, and reaction was carried out at room temperature for one hour. After phenol extraction and chloroform extraction, about 6.0 µg of DNA fragments was recovered by ethanol precipitation. 6.0 µg of the DNA fragments was dissolved in 120 µℓ (total volume) of Y-100 buffer. 12 units of BamHI was added and cleavage reaction was carried out at 37°C for 3 hours. About 0.1 µg of DNA fragments [BglII (partial cleavage)-Klenow fragment-BamHI fragments] of about 950 bp containing the whole region of UL-C polypeptide except a part of the N-terminal region was obtained from the reaction solution by LGT method.

Separately, 2 µg of pGEL1 was dissolved in 40 µℓ of Y-100 buffer. 4 units each of restriction enzymes HindIII, PstI and BamHI were added and cleavage reaction was carried out at 37°C for 3 hours. About 0.7 µg of DNA fragments (PstI-BamHI fragments) of about 1.7 Kb containing the terminator of lipoprotein was obtained from the reaction solution by LGT method.

3 µg of pKYP10 prepared by the method described in Japanese Published Unexamined Patent Application No. 110600/83 was dissolved in 60 µℓ of Y-100 buffer. 6 units each of restriction enzymes BanIII (product of Toyobo) and PstI were added and cleavage reaction was carried out at 37°C for 3 hours. About 0.6 µg of DNA fragments (BanIII-PstI fragments) of about 1.1 Kb containing tryptophan promoter (Ptrp) was obtained from the reaction solution by LGT method.

Separately, the following DNA linker was synthesized in order to furnish the sequence from the N-terminal Met (ATG) to the second base (CA) of the second amino acid Gln (CAG) of the physiologically active polypeptide (UL-C) and to adjust the distance between the SD sequence downstream from Ptrp and the ATG to an appropriate length of 6 to 18 bp.

```
     BanIII              15-mer  Met     blunt end
 5'-|C G A T A A G C T T |A T G|C A |-3'
    3'-|T A T T C G A A T A C G T |-5'
                13-mer
```

First, two single stranded DNAs of 13-mer and 15-mer were synthesized by the conventional triester method. Then, 20 pmoles each of the 13-mer and 15-mer DNAs were dissolved in 40 µℓ (total volume) of a solution containing 50 mM Tris-HCl (pH 7.5), 10 mM MgCl$_2$, 5 mM dithiothreitol, 0.1 mM EDTA and 1 mM ATP. One unit of T4 polynucleotide kinase (product of Takara Shuzo) was added and phosphorylation reaction was carried out at 37°C for 60 minutes.

0.1 µg of the BglII (partial cleavage)-Klenow fragment-BamHI fragments (about 950 bp) obtained from pLGC:Scll as above, 0.1 µg of the PstI-BamHI fragments (about 1.7 Kb) obtained from pGELl as above and 0.3 µg of the BanIII-PstI fragments (about 1.1 Kb) obtained from expression vector pKYP10 as above were dissolved in 20 µℓ of T4 ligase buffer, and about 1 pmole of the DNA linker mentioned above was added to the mixture. 50 units of T4 DNA ligase was added to the mixture and reaction was carried out at 4°C for 18 hours.

_Escherichia_ _coli_ C600 SF8 strain was transformed using the reaction mixture containing recombinant plasmids and Ap$^r$ colonies were obtained. Plasmid DNA was recovered from the cultured cells of the colonies. The structure of the thus obtained plasmid was confirmed by polyacrylamide gel electrophoresis after cleavage with restriction enzymes EcoRI, BglII and BamHI. The plasmid was named pLGC-xl. It was confirmed by the dideoxy sequence method that the nucleotide sequence around the HindIII site of pLGC-xl was as follows:

```
    BanIII    HindIII      Met   Gln   Ile   Phe   Val
    |C G A T A|A G C T|T A T G C A G A T C T T C G T G
            ↓         ↓
```

Example 11

Production of the physiologically active polypeptide (UL-C) by _Escherichia_ _coli_ carrying pLGC-xl:

_Escherichia_ _coli_ C600 SF8 strain carrying recombinant plasmid pLGC-xl obtained in Example 10 [C600 SF8 strain carrying pLGC-xl was named ELGC-xl] was cultured in LG medium at 37°C for 18 hours. 0.5 mℓ of the culture liquor was inoculated into 10 mℓ of MCG medium containing 25 µg/mℓ tryptophan and 50 µg/mℓ ampicillin, and culturing was carried out at 30°C for 4 - 8 hours. Then, IAA which is a tryptophan-operon-inducing substance was added thereto to make a concentration of 10 µg/mℓ, and culturing was continued for 2 - 12 hours. The culture broth was centrifuged at 8,000 rpm for 10 minutes and collected cells were washed with PBS. The washed cells were suspended in 5 mℓ of PBS and subjected to ultrasonic disruption (Branson Sonic Power Company, Sonifier Cell Disruptor 200, output control 2) at 0°C for 10 minutes. The disrupted cells were centrifuged at 15,000 rpm for 30 minutes to extract the physiologically active polypeptide into the supernatant. After the cells were removed with a 0.45 µm filter (product of Nihon Millipore, Limited) and the cell growth-promoting activity thereof was determined according to the process described in Example 3. The results are shown in Table 7.

Table 7

| Plasmid | Propagation rate (2) |
|---------|----------------------|
| pLGC-xl | 1.48 |
| pcDV1 (1) | 1.00 |

(1) A cell extract solution was prepared from C600 SF8 strain carrying plasmid (pcDV1) which does not contain the DNA coding for the physiologically active polypeptide in the same manner as described above, and assayed.

(2) Propagation rate is relative to the propagation obtained in (1) as 1.00.

Example 12

Construction of recombinant plasmid pSEA-el:

Escherichia coli C600 SF8 strain (EAGE103) carrying pAGE103 (4.1 Kb) was cultured, and pAGE103 DNA was prepared from the cultured cells by a conventional method. 2 μg of the thus obtained pAGE103 DNA was dissolved in 30 μℓ of Y-100 buffer. 4 units each of HindIII and BamHI were added and cleavage reaction was carried out at 37°C for 2 hours. About one μg of DNA fragments (HindIII-BamHI fragments) of 4.1 Kb containing SV40 early promoter ($P_{SE}$) was obtained from the reaction solution by LGT method.

Separately, Escherichia coli C600 SF8 strain (ELGA-el) carrying pLGA-el (3.3 Kb) was cultured, and pLGA-el DNA was prepared from the cultured cells by a conventional method. 3 μg of the thus obtained pLGA-el DNA was dissolved in 40 μℓ of Y-100 buffer. 6 units each of HindIII and BamHI were added and cleavage reaction was carried out at 37°C for 2 hours. About 0.2 μg of DNA fragments (HindIII-BamHI fragments) of about 560 bp containing the physiologically active polypeptide gene was obtained from the reaction solution by LGT method.

Then, 0.5 μg of the HindIII-BamHI fragments (about 4.1 Kb) obtained from pAGE103 as above and 0.1 μg of the HindIII-BamHI fragments (about 560 bp) obtained from pLGA-el as above were dissolved in 20 μℓ (total volume) of T4 ligase buffer. 2 units of T4 DNA ligase was added and ligation reaction was carried out at 4°C for 18 hours.

Escherichia coli C600 SF8 strain was transformed using the reaction mixture containing recombinant plasmids and kanamycin resistant colonies were obtained. Plasmid DNA was recovered from the cultured cells of the colonies and pSEA-el was obtained. The structure of pSEA-el was confirmed by agarose gel electrophoresis after cleavage with HindIII, XhoI, PvuII and BanIII.

Example 13

Production of the physiologically active peptide by
animal cells carrying pSEA-el:

Introduction of pSEA-el and pSV2-dhfr into CHO
strain deficient in dhfr was carried out according to calcium
phosphate method. That is, cells were inoculated in an amount
of $1 \times 10^5$ cells/ml into 5 ml of MEM ALPHA medium (containing
ribonucleic acids and deoxyribonucleic acids; product of Gibco
Oriental) which further contains 10% FCS, 7.5% $NaHCO_3$ solution
(product of Flow Laboratory Inc.) in an amount of 1/50 and
100 x non-essential amino acids solution (product of Flow
Laboratory Inc.) in an amount of 1/100 [referred to as MEMα
(non selection medium) hereinafter]. Culturing was carried
out in $CO_2$ incubator at 37°C for one day using a dish with a
diameter of 6 cm (product of LUX; dishes produced by LUX were
used for the culturing hereinafter). Separately, 10 μg of
pSEA-el DNA and 1 μg of pSV2-dhfr DNA were dissolved in 450 μl
of 10 mM Tris-HCl (pH 7.5) solution. To the solution was
added 500 μl of a solution containing 280 mM NaCl, 1.5 mM
$Na_2HPO_4$ and 50 mM HEPES (pH 7.1). Further, 50 μl of 2.5M
$CaCl_2$ solution was added to the mixture and the whole mixture
was allowed to stand at room temperature for 5 minutes. The
whole DNA solution was added to CHO strain deficient in dhfr
which was obtained by further culturing in 10 ml of fresh MEMα
(non selection medium) for one hour, and incubation was
carried out for 8 hours. After the cells were washed with
PBS, 5 ml of MEMα (non selection medium) was added and
culturing was carried out for 16 hours. The cells were washed
with PBS, and 3 ml of a solution containing 0.05% trypsin and
0.02% EDTA (ethylene diamine tetraacetate) was added. After
the removal of excess solution, incubation (trypsin treatment)
was carried out at 37°C for 5 minutes. MEM ALPHA medium (free
of ribonucleic acids and deoxyribonucleic acids) containing
10% dialyzed FCS (product of Gibco Oriental), 7.5% $NaHCO_3$
solution in an amount of 1/50, 100 x non-essential amino acids solution in an amount of 1/100 and 0.3 mg/mℓ G418 (product of Gibco Oriental) [referred to as MEMα (selection medium) hereinafter] was added and the cells were suspended. The cells were then cultured in $CO_2$ incubator at 37°C for 5 days using a dish with a diameter of 10 cm. After the cells were washed with PBS, MEMα (selection medium) was added and culturing was continued for 5 days. The same operation was repeated and culturing was carried out for further 5 days. The cells were washed with PBS and subjected to trypsin treatment. Then, 10 mℓ of MEMα (selection medium) was added and the cells were suspended. The cells were cultured in $CO_2$ incubator at 37°C for 7 days using a dish with a diameter of 10 cm. After trypsin treatment, the cells were inoculated into a dish with a diameter of 10 cm in a cell concentration of $5 \times 10^5 - 1 \times 10^6$/mℓ using 10 mℓ of MEMα (selection medium). The cells were subjected to trypsin treatment after culturing for 4 days and inoculated into a dish with a diameter of 15 cm containing 12 mℓ of MEMα (selection medium) to continue culturing for 4 days. Then, the cells were washed and suspended in 0.5 mℓ of PBS. After disruption with Teflon homogenizer, the cells were centrifuged at 12,000 rpm for 20 minutes. The thus obtained supernatant was subjected to the determination of activity. The determination of cell growth-promoting activity was carried out according to the method described in Example 3. The results are shown in Table 8.

Table 8

| Plasmid | Propagation rate (2) |
|---|---|
| pSEA-el & pSV2-dhfr | 1.87 |
| pAGE103 & pSV2-dhfr (1) | 1.00 |

(1) A cell extract solution was prepared from the transformant obtained using plasmid (pAGE103) which does not contain the DNA coding for the physiologically active polypeptide in the same manner as described above, and assayed.

(2) Propagation rate is relative to the propagation obtained in (1) as 1.00.

Reference Example 1

Erythroleukemia cell strain K562-Tl was put into a large spinner flask (20 ℓ) containing 8 ℓ of the medium having the following composition at a concentration of $8 \times 10^5$ cells/mℓ, and cultured at 37°C for 3 days. The medium was a protein-free medium prepared by mixing Ham F10 medium (made by Flow Laboratory Inc.) and Dulbecco's Modified Eagle (DME) medium (made by Nissui Seiyaku) in a ratio of 3:1 to make a basal medium, and adding pyruvic acid (5 mM), selenious acid ($1.25 \times 10^{-7}$ M), galactose (1 mg/mℓ), glutamine (4 mM), penicillin (25 U/mℓ), streptomycin (25 µg/mℓ) and sodium bicarbonate (0.01%) to the basal medium. Then, 120 ℓ of the culture medium was concentrated to about 100 mℓ through a hollow fiber (molecular weight cut: 3,000; made by Amicon Co.), and about 100 mℓ of the concentrate was dialyzed against 1% acetic acid at 4°C for 24 hours. The resulting precipitates were removed by centrifugation at 12,000 rpm for 10 minutes, and the supernatant was freeze-dried to remove acetic acid. The obtained powder was dissolved in 120 mℓ of distilled water.

The thus obtained solution was passed through a column of 160 mℓ of QAE-Sephadex-A5 (made by Pharmacia Fine Chemicals Inc.) in two 60 mℓ portions. Then, elution was carried out with 150 mℓ of PBS, and further with 190 mℓ of 0.1% acetic acid, whereby 300 mℓ of the active fractions eluted with PBS was obtained. The activity recovery was about 8.3%, and the specific activity was 1.9-fold increased.

Then, 300 mℓ of the active fractions was freeze-dried, and the obtained powder was dissolved in 20 mℓ of distilled water. The solution was passed through a column containing 400 mℓ of Biogel P-60 at SV5.0 in two portions. Elution was carried out with PBS, and the eluate was collected in 6 mℓ fractions, whereby 60 mℓ of the 41st to 45th fractions was obtained. The activity recovery was 1.7%, and the specific activity was 13.3-fold increased. The active fractions were passed through a column containing 5 mℓ of FPLC (fast protein liquid chromatography) Mono S (made by Pharmacia Fine Chemicals Inc.). The column was washed with PBS and eluted with an aqueous 0 - 0.5 M sodium chloride solution. The eluate was separated into 1 mℓ fractions, and the activity was observed in the 53rd fraction. The activity recovery was 0.2%, and the specific activity was 3,600-fold increased.

The results of the above purification process are given in Table 5.

Table 5

| | Volume mℓ | $OD_{280}$ OD/mℓ | Activity unit | Total $OD_{280}$ | Specific activity $OD_{280}$ | Purification effect |
|---|---|---|---|---|---|---|
| After dialysis against acetic acid | 120 | 410 | 54000 | 4800 | 11.25 | x 1 |
| QAE-Sephadex eluate | 300 | 0.7 | 4500 | 210 | 21.43 | x 1.9 |
| Biogel P-60 eluate | 150 | 0.1 | 900 | 6 | 150 | x 13.3 |
| FPLC eluate | 3 | 0.001 | 120 | 0.003 | 40000 | x 3600 |

The amino acid sequence from the N-terminal amino acid to the 30th amino acid of the polypeptide was determined by a combination of Type 470A sequencer made by Applied Biosystems with high pressure liquid chromatography made by Spectra Physics.   The sequence was found as follows:

Met-Gln-Ile-Phe-Val-Lys-Thr-Leu-Thr-Gly-Lys-
Thr-Ile-Thr-Leu-Glu-Val-Glu-Pro-X-Asp-X-Ile-
X-Asn-Val-X-Ala-X-Ile-
(X shows unidentified amino acids)

What is Claimed is:

1.  A polypeptide having a polypeptide sequence selected from the peptide sequences shown in Tables 2, 3 and 6.

2.  A proteinaceous substance containing a polypeptide having the peptide sequence shown in Table 3 as a part of the structure.

3.  A polypeptide according to Claim 1 or 2 for use as a growth promoting agent upon human B lymphocytic cells.

4.  A DNA coding for a polypeptide having a peptide sequence selected from the peptide sequences shown in Tables 2, 3 and 6.

5.  A recombinant DNA wherein a DNA coding for a polypeptide having a peptide sequence selected from the peptide sequences shown in Tables 2, 3 and 6 is incorporated.

6.  The recombinant DNA according to Claim 5, which is named plasmid pLGA8, pLGB12 or pLGC15.

7.  A microorganism or an animal cell containing a recombinant DNA wherein a DNA coding for a polypeptide having a peptide sequence selected from the peptide sequences shown in Tables 2, 3 and 6 is incorporated.

8.  The microorganism according to Claim 7, wherein the microorganism belongs to Escherichia coli.

9.  The microorganism according to Claim 8, wherein the microorganism is Escherichia coli ELGA8, Escherichia coli ELGB-al or Escherichia coli ELGC15.

10. A process for producing a polypeptide, characterized by culturing in a medium a microorganism or an animal cell containing a recombinant DNA wherein a DNA coding for a polypeptide having a peptide sequence selected from the peptide sequences shown in Tables 2, 3 and 6 is incorporated, accumulating the polypeptide in a culture broth, and recovering the polypeptide therefrom.

11. The process according to Claim 10, characterized in that a plasmid DNA having a tryptophan promoter or SV40 early promoter is used as the plasmid DNA and the DNA fragment is inserted downstream from the promoter of the plasmid DNA.

12. The process according to Claim 10 or 11, wherein the microorganism belongs to _Escherichia_ _coli_.

13 Medicament containing as active ingredient a polypeptide according to Claim 1 or 2.

14 Pharmaceutical composition comprising a polypeptide according to Claim 1 or 2 and a pharmaceutically acceptable carrier.

15 Pharmaceutical composition comprising a polypeptide according to Claim 1 or 2 and a pharmaceutically acceptable carrier to be used as an immunoactivating or carcinostatic agent.

16 Use of a polypeptide or composition according to Claim 1 or 2 and Claim 14, respectively, for the manufacture of a medicament having a growth promoting activity upon human B lymphocytic cells.

17 Use of a polypeptide or composition according to Claim 1 or 2 and 14, respectively, for the manufacture of a medicament to be used as an immunoactivator or carcinostatic agent.

# FIG. 1 (1)

## FIG. 1 (2)

## FIG.2

pLGA 8
(pLGA 9,11,13)

XhoI  EcoRI  PvuII  AccI

pLGB 12
(pLGB 16,19)

BanII  PvuII  SalI  XmnI

pLGC 15

BamHI  BglII  SalI  BglII  SalI  BglII  SalI
PvuII  PvuII  PvuII

## FIG.3

pLGB12 (circular plasmid)
- HpaII
- HpaII
- HpaII
- Ap$^r$
- HpaII
- BamHI
- HpaII
- UL-B
- BamHI
- HpaII
- HpaII
- HpaII
- HpaII

HpaII → BamHI → 490bp

pKYP10 (circular plasmid)
- Ptrp
- HindIII
- BamHI
- Ap$^r$
- Tc$^r$

HindIII + BamHI → 4.4Kb

### 31-mer

|  | Met | Gln | Ile | Phe | Val | Lys | Thr | Leu |  |
|--|-----|-----|-----|-----|-----|-----|-----|-----|--|
| 5'-AGCT | T ATG | CAA | ATA | TTC | GTA | AAG | ACG | TTA | AC-3' |
| 3'- A | TAC | GTT | TAT | AAG | CAT | TTC | TGC | AAT | TG GC-5' |

29-mer

Kination (kinase reaction)

T4 DNA ligase

pLGB-α1 (circular plasmid)
- PvuII
- Ptrp
- pvuII
- Ap$^r$
- UL-B
- PstI
- BamHI
- PvuII

# FIG.4

# FIG.5

## FIG.6

# FIG.7

# FIG.8

FIG.9

0249854

European Patent Office

Application number:

87108303.6

●

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

Accession numbers of the deposits:
FERM BP 1067
FERM BP 1068
FERM BP 1070
FERM BP 1312
FERM BP 1313

EPO Form 1165 07.81 e